# FASCICULE DE BREVET EUROPEEN

(11) **EP 0 520 879 B1**
(45) Date de publication et mention de la délivrance du brevet: **23.04.1997**
(21) Numéro de dépôt: 92401764.3
(22) Date de dépôt: 24.06.1992
(51) Int. Cl.: C07J 31/00, C07J 41/00

(54) **Nouveaux stéroides 16-méthyl substitués dérivés de la pregna 1,4-diène 3,20-dione, leur préparation, leur application à la préparation de steroides 16-méthylène et nouveaux intermédiaires**
Neue 16-Methyl substituierte Steroidderivate von Pregna-1,4-dien-3,20-dion, ihre Herstellung, ihre Verwendung zur Herstellung von 16-Methylene-substituierte Steroide und neue Zwischenprodukten dafür
New 16-methyl substituted steroid derivatives of pregna-1,4-dien-3,20-dione, their preparation, their use in the preparation of 16-methylene substituted steroids and new intermediates therefor

(30) Priorité: 25.06.1991 FR 9107784
(43) Date de publication de la demande: 30.12.1992
(73) Titulaire: ROUSSEL UCLAF, 93230 Romainville (FR)
(72) Inventeur: Boivin, Jean, F-91470 Forges les Bains (FR); Chauvet, Christine, F-75015 Paris (FR); Zard, Samir, F-91190 Gif sur Yvette (FR)
(74) Mandataire: Vieillefosse, Jean-Claude

(56) Documents cités:
- EP-A- 0 104 054
- EP-A- 0 148 616
- JOURNAL OF CHEMICAL RESEARCH (S), no.11, 1985 pages 344 - 345 E. HATZIGRIGORIOU ET AL 'Selective Functionalisation of the 16-Position in 16-Dehydroprogesterones: Synthesis of 16-alpha-Acyl and -Cyanomethyl Derivatives'

## Description

La présente invention a pour objet des nouveaux stéroides 16-méthyl substitués dérivés de la pregna 1,4-diène-3,20-dione, leur préparation, leur application à la préparation de stéroides 16-méthylène et des nouveaux intermédiaires.

L'invention a ainsi pour objet les composés de formule (I) : 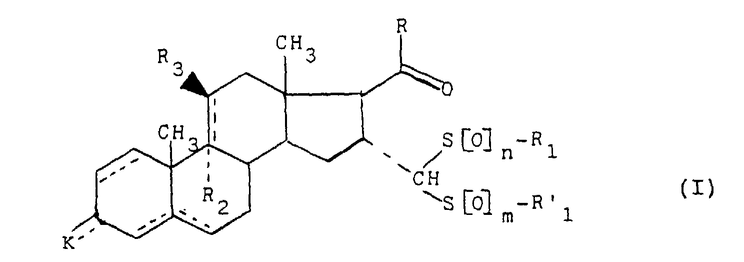 dans laquelle 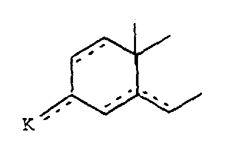 représente soit un système 3-céto Δ4 soit un système 3-céto Δ1,4 soit un système 3-OR_{**4**} Δ5 dans lequel R_{**4**} représente un atome d'hydrogène ou un groupement protecteur du radical hydroxy, R représente un radical méthyle, un radical -CH_{**2**}OH ou un radical -CH_{**2**}-OR', dans lequel R' représente un groupement protecteur du radical hydroxy, R_{**1**} et R'_{**1**}, identiques ou différents, représentent un radical méthyle, un radical alkyle ramifié ne possédant pas d'atome d'hydrogène en position béta, renfermant de 5 à 8 atomes de carbone, un radical aryle renfermant jusqu'à 10 atomes de carbone, un radical hétéroaryle renfermant jusqu'à 10 atomes de carbone et un ou plusieurs hétéroatomes choisis parmi l'azote, le soufre et l'oxygène, ou un radical benzyle, n et m, identiques ou différents, représentent les nombres 0 ou 1, R_{**2**} et R_{**3**} représentent un atome d'hydrogène ou R_{**2**} représente un atome de fluor et R_{**3**} représente un radical formyloxy ou acétyloxy et les traits pointillés en position 9(11) symbolisent la présence éventuelle d'une seconde liaison.

R' peut notamment représenter un radical alkyle renfermant de 1 à 6 atomes de carbone et plus particulièrement un radical méthyle, éthyle, propyle linéaire ou ramifié ou butyle linéaire ou ramifié.

R' peut encore notamment représenter un radical acyle renfermant de 1 à 6 atomes de carbone et plus particulièrement un radical formyle, acétyle, propionyle, butyryle ou pivaloyle.

R' peut encore notamment représenter un radical aralkyle notamment benzyle, un radical tétrahydropyrannyle, un radical alkyl, aryl ou arylalkyl silyle, notamment triméthyl silyle, tert-butyl diméthyl silyle, triphényl silyle ou diphényl tert-butyl silyle.

La liste des valeurs ci-dessus pour R' ne présente pas un caractère limitatif, tout groupement protecteur du radical hydroxy connu de l'homme du métier compatible avec les conditions opératoires du procédé défini ci-après peut convenir.

Lorsque R_{**1**} et R'_{**1**} représentent un radical alkyle renfermant de 5 à 8 atomes de carbone, il s'agit d'un radical néopentyle ou d'un homologue supérieur ne possédant pas d'atome d'hydrogène en position béta.

Lorsque R_{**1**} et R'_{**1**} représentent un radical aryle, il s'agit notamment d'un radical phényle, d'un radical phényle substitué par un ou deux radicaux méthyles ou d'un radical naphtyle.

Lorsque R_{**1**} et R'_{**1**} représentent un radical hétéroaryl, il s'agit notamment d'un radical pyridyle, thiazolyle ou benzothiazolyle.

Lorsque R_{**4**} représente un groupement protecteur du radical hydroxy, il s'agit notamment de l'un des groupements cités ci-dessus pour R'.

Parmi les produits de formule (I), on peut citer les produits de formule (I') : 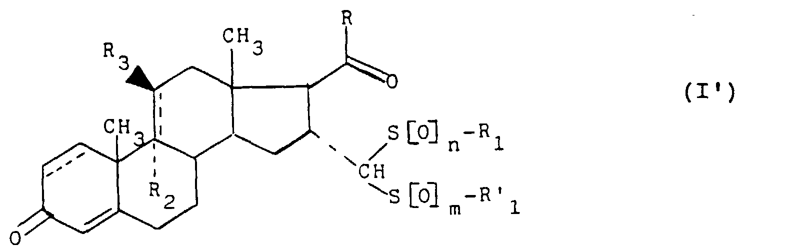 dans laquelle R, R_{**1**}, R'_{**1**}, R_{**2**}, R_{**3**}, m et n ont la signification indiquée ci-dessus et les traits pointillés en 1(2) et 9(11) symbolisent la présence éventuelle d'une seconde liaison.

L'invention a plus particulièrement pour objet les composés de formule (I), telle que définie précédemment, dans laquelle R représente un radical méthyle, un radical -CH_{**2**}OH ou un radical -CH_{**2**}OR" dans lequel R" représente un radical alkyle renfermant de 1 à 4 atomes de carbone, un radical acyle renfermant de 1 à 5 atomes de carbone ou un radical benzyle, R_{**1**} et R'_{**1**}, identiques ou différents, représentent un radical méthyle, phényle, tolyle ou benzyle, n, m, R_{**2**}, R_{**3**} et les traits pointillés étant définis comme ci-dessus.

Parmi les composés de formule (I) tels que définis précédemment, l'invention a ainsi pour objet :
- les composés de formule (I), tels que définis précédemment, répondant à la formule (I_{**A**}) : 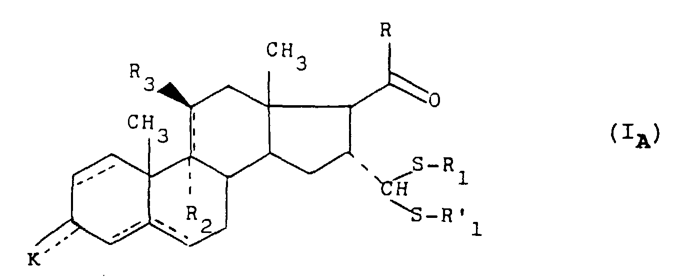 dans laquelle K, R, R_{**1**}, R'_{**1**}, R_{**2**}, R_{**3**} et les traits pointillés sont définis comme précédemment,
- les composés de formule (I), tels que définis précédemment, répondant à la formule (I_{**B**}) : 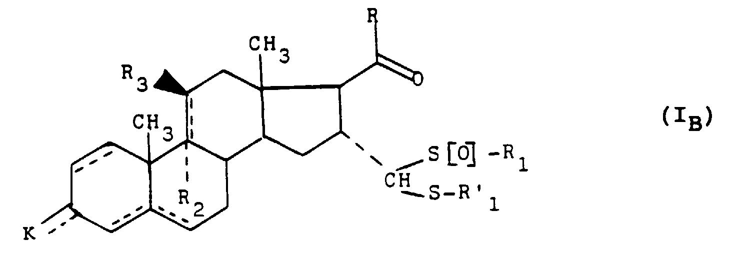 dans laquelle K, R, R₁, R'₁, R₂, R₃ et les traits pointillés sont définis comme précédemment ;
- les composés de formule (I), tels que définis précédemment, répondant à la formule (I_{c}) : 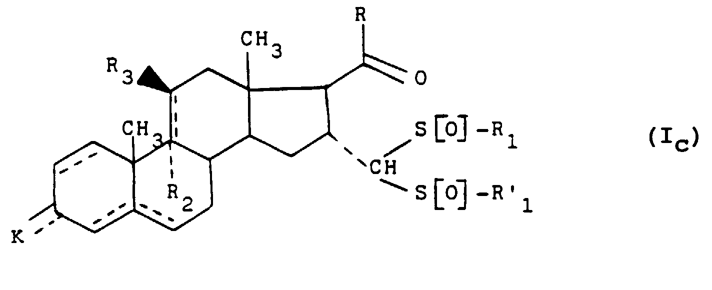 dans laquelle K, R, R₁, R'₁, R₂, R₃ et les traits pointillés sont définis comme précédemment.

Parmi les composés de formule (I), objet de l'invention, on peut citer également ceux caractérisés en ce que 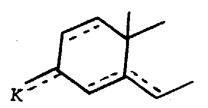 représente un système 3-céto Δ1,4 et R représente un radical -CH₂-OR", R" étant défini comme précédemment, R₃ représente un atome d'hydrogène et les traits pointillés en position 9(11), représentent une seconde liaison.

Parmi les composés de formule (I), l'invention a tout particulièrement pour objet :
- la 21-acétoxy 16alpha-[bis(phénylthio)-méthyl] pregna-1,4,9(11)triène-3,20-dione ;
- la 21-acétoxy 16alpha-[(phénylthio) (phénylsulfinyl)-méthyl] pregna-1,4,9(11)-triène-3,20-dione ;
- la 21-acétoxy 16alpha-[bis(phénylsulfinyl)-méthyl] pregna-1,4,9(11)-triène-3,20-dione.

L'invention a également pour objet un procédé de préparation des composés de formule (I), telle que définie plus haut, caractérisé en ce que l'on soumet un composé de formule (II) : 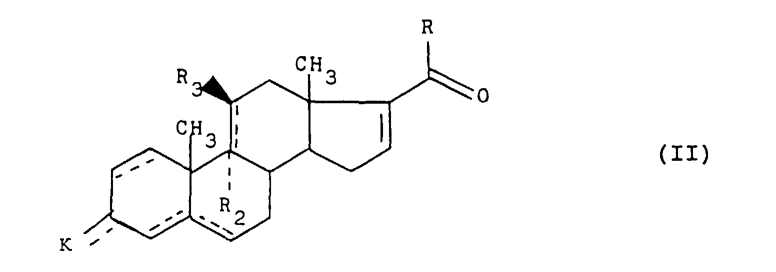 dans laquelle K, R, R_{**2**}, R_{**3**} et les traits pointillés ont la signification indiquée plus haut, en milieu basique, à l'action d'un réactif de formule (P) :

R₁S-CH₂-NO₂ (P)

dans laquelle R_{**1**} a la signification indiquée plus haut, pour obtenir un composé de formule (III) : 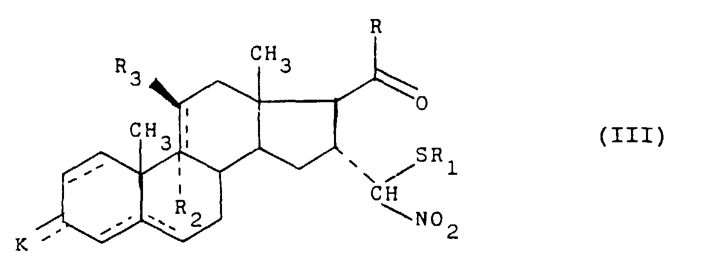 dans laquelle K, R, R_{**1**}, R_{**2**}, R_{**3**} et les traits pointillés ont la signification indiquée plus haut, que l'on soumet, en présence d'un acide, à l'action d'un thiol ou d'un thiophénol de formule :

HS-R'₁

dans laquelle R'_{**1**} a la signification précitée, pour obtenir un composé de formule (I_{**A**}) : 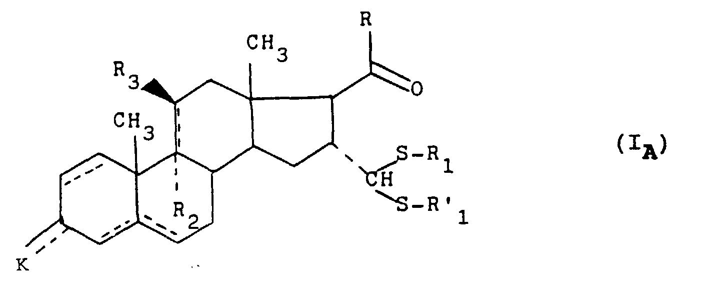 dans laquelle K, R, R_{**1**}, R'_{**1**}, R_{**2**}, R_{**3**} et les traits pointillés ont la signification précitée, correspondant à un composé de formule (I) dans laquelle m et n sont égaux à 0, composé de formule (I_{**A**}) que, le cas échéant, l'on soumet soit à l'action d'un équivalent d'un agent oxydant, pour obtenir un composé de formule (I_{**B**}) : 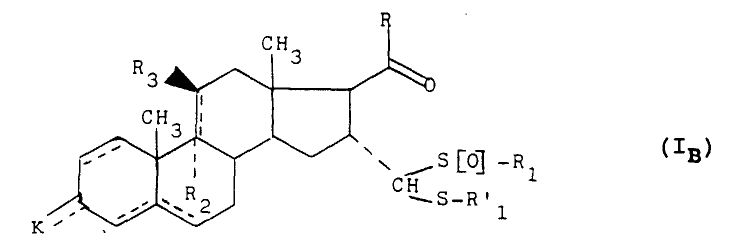 dans laquelle K, R, R_{**1**}, R'_{**1**}, R_{**2**}, R_{**3**} et les traits pointillés ont la signification précitée, correspondant à un composé de formule (I) dans laquelle l'une des valeurs m et n est égale à 1 et l'autre à 0, soit à l'action d'au moins deux équivalents d'un agent oxydant, pour obtenir un composé de formule (I_{**c**}) : 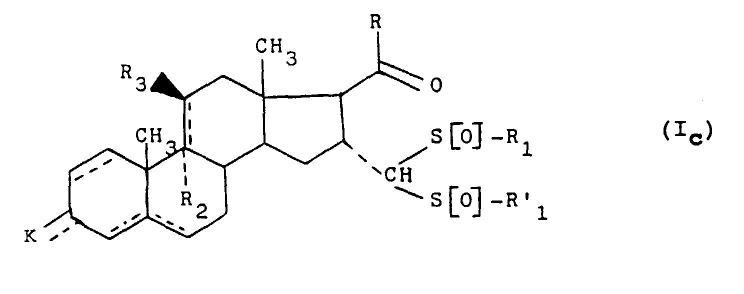 dans laquelle K, R, R_{**1**}, R'_{**1**}, R_{**2**}, R_{**3**} et les traits pointillés ont la signification précitée, correspondant à un composé de formule (I) dans laquelle m et n sont égaux à 1.

L'invention a notamment pour objet un procédé selon ce qui précède, caractérisé en ce que l'on soumet un composé de formule (II) : 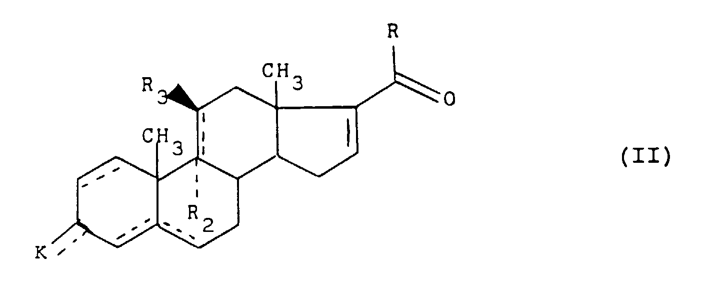 dans laquelle K, R, R_{**2**}, R_{**3**} et les traits pointillés ont la signification indiquée plus haut, en milieu basique, à l'action d'un réactif de formule (P) :

R₁S-CH₂-NO₂ (P)

dans laquelle R_{**1**} a la signification indiquée plus haut, pour obtenir un composé de formule (III) : 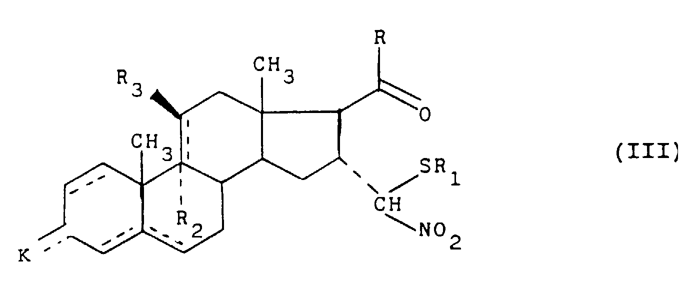 dans laquelle K, R, R_{**1**}, R_{**2**}, R_{**3**} et les traits pointillés ont la signification indiquée plus haut, que l'on soumet en présence d'un acide à l'action d'un thiol ou d'un thiophénol de formule :

HS-R'₁

dans laquelle R'_{**1**} a la signification précitée, pour obtenir un composé de formule (I_{**A**}) : 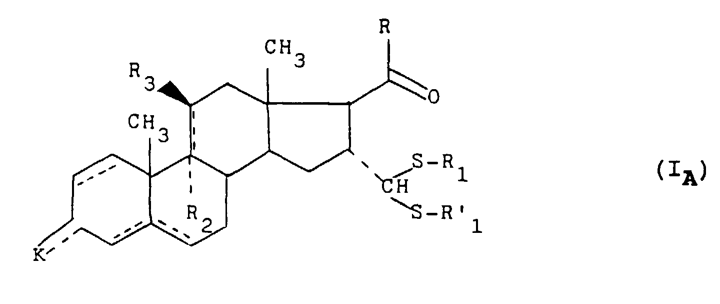 dans laquelle K, R, R_{**1**}, R'_{**1**}, R_{**2**}, R_{**3**} et les traits pointillés ont la signification précitée, correspondant à un composé de formule (I) dans laquelle m et n sont égaux à 0, composé de formule (I_{**A**}) que l'on soumet à l'action d'au moins deux équivalents d'un agent oxydant, pour obtenir un composé de formule (I_{**c**}) : 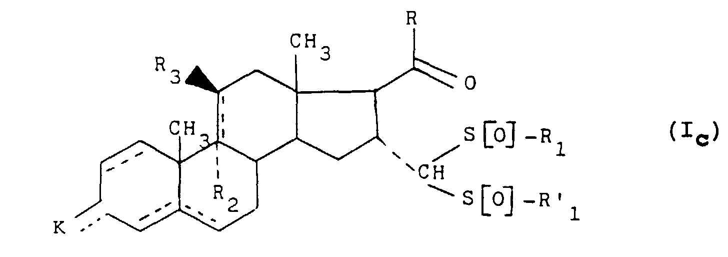 dans laquelle K, R, R_{**1**}, R'_{**1**}, R_{**2**}, R_{**3**} et les traits pointillés ont la signification précitée, correspondant à un composé de formule (I) dans laquelle m et n sont égaux à 1.

L'action du réactif de formule P sur le composé de formule (II) est effectuée en présence d'une base qui peut être notamment une base aminée, par exemple une amine secondaire ou tertiaire telle que la diéthylamine ou la triéthylamine, le diazabicycloundécène (DBU), le diazabicyclononène (DBN), un acétate, un carbonate, un hydrure, un hydroxyde ou un alcoxyde alcalin, les bases fortes étant utilisées de préférence en quantité catalytique.

On opère au sein d'un solvant organique qui peut être par exemple un éther tel que le tétrahydrofuranne ou le dioxanne, un alcool tel que le méthanol ou l'éthanol, un solvant aromatique tel que le benzène ou le toluène, ou encore le diméthylformamide, le diméthylsulfoxyde ou le chlorure de méthylène.

L'action du thiol ou du thiophénol est catalysée par un acide de préférence faible, lequel peut notamment être un acide carboxylique, tel que l'acide formique, acétique ou propionique, un acide de Lewis tel que le chlorure de zinc ou le chlorure d'aluminium, ou encore l'acide phosphorique. Le dit acide peut aussi, le cas échéant, jouer le rôle de solvant. Le solvant peut encore être un solvant aromatique tel que le benzène ou le toluène. Il va de soi que les conditions de la réaction sont choisies le cas échéant de manière telle, qu'elles soient compatibles avec le groupement protecteur du radical hydroxy que le composé (II) peut porter et, plus généralement, avec la structure de la molécule, que l'on souhaite préserver. Ceci est connu de l'homme du métier.

L'agent oxydant utilisé pour transformer le composé de formule (I_{A}) en monosulfoxyde ou en disulfoxyde peut être un peracide tel que l'acide m-chloro perbenzoïque, l'acide perbenzoïque, l'acide perphtalique, le sel de magnésium de l'acide monoperphtalique, ou encore l'eau oxygénée en présence d'un acide carboxylique tel que l'acide acétique, ou encore un périodate, un perborate ou un persulfate, notamment de sodium ou de potassium.

L'invention a aussi pour objet, à titre de composés industriels, et notamment à titre d'intermédiaires utiles dans la préparation des composés de formule (I), les composés de formule (III), telle que définie plus haut.

L'invention a également pour objet l'application des composés de formule (I), telle que définie plus haut, à la préparation des composés de formule (A) : 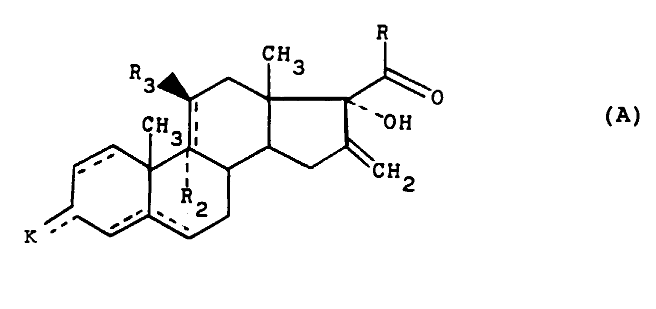 dans laquelle K, R, R_{**2**}, R_{**3**} et les traits pointillés ont la signification indiquée plus haut, caractérisée en ce que :
- ou bien l'on soumet un composé de formule (I_{**A**}) telle que définie plus haut, à l'action d'au moins deux équivalents d'un agent oxydant, pour obtenir un composé de formule (I_{**c**}) telle que définie plus haut, que l'on soumet, à chaud, à l'action d'un agent thiophile, pour obtenir le composé de formule (A) attendu ;
- ou bien l'on soumet un composé de formule (I_{**c**}) telle que définie plus haut, à chaud, à l'action d'un agent thiophile, pour obtenir le composé de formule (A) attendu ;
- ou bien l'on chauffe un composé de formule (I_{**B**}) telle que définie plus haut, le cas échéant en présence d'un agent thiophile, pour obtenir un composé de formule (IV) : 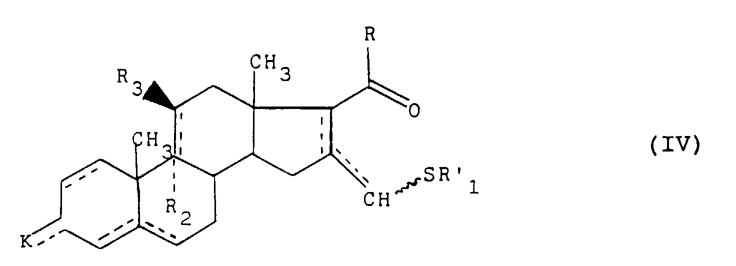 dans laquelle K, R, R'_{**1**}, R_{**2**}, R_{**3**} et les traits pointillés ont la signification indiquée plus haut, le trait pointillé en 16(17) et 16(16') symbolise l'existence d'un mélange de sulfure vinylique et allylique et le trait ondulé symbolise l'existence d'un mélange d'isomères, composé de formule (IV) que l'on soumet à l'action d'au moins un équivalent d'un agent oxydant, pour obtenir un composé de formule (V) : 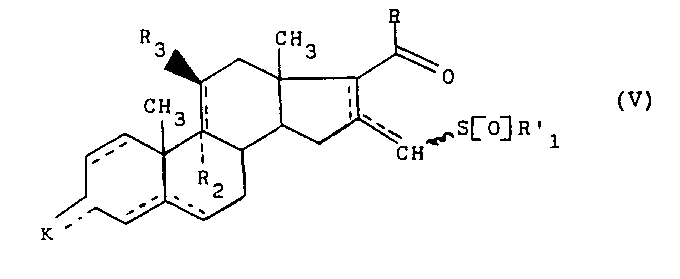 dans laquelle K, R, R'_{**1**}, R_{**2**}, R_{**3**} les traits pointillés et le trait ondulé ont la signification indiquée ci-dessus, que l'on soumet, à chaud, à l'action d'un agent thiophile, pour obtenir le composé de formule (A) attendu.

Dans l'application selon l'invention, on peut :
- isoler ou ne pas isoler le disulfoxyde de formule (I_{**c**}) ;
- éliminer d'abord, par chauffage seulement, un premier groupement -S[O]R_{**1**} sur le disulfoxyde de formule (I_{**c**}), pour obtenir le composé monosulfoxyde de formule (V) telle que définie plus haut, puis éliminer le second groupement -S[O]R_{**1**}, via la forme sulfénate intermédiaire décrite plus loin, par action, à chaud, d'un agent thiophile ;
- isoler ou ne pas isoler le monosulfoxyde de formule (V) ainsi obtenu ;
- éliminer par chauffage seulement le groupement -S[O]R_{**1**} sur le composé de formule (I_{**B**}) ;
- isoler ou ne pas isoler le monosulfoxyde de formule (V) obtenu par oxydation du composé de formule (IV).

L'agent oxydant utilisé dans l'application selon l'invention pour transformer le composé de formule (I_{**A**}) en un composé de formule (I_{**c**}), ou pour transformer le composé de formule (IV) en un composé de formule (V) est l'un de ceux qui ont été cités plus haut.

L'agent thiophile peut être par exemple la triphénylphosphine, la triméthyl ou triéthylphosphine, le triphényl, triméthyl ou triéthylphosphite, le diméthyl ou diéthylphosphite, une amine secondaire, par exemple la diéthylamine, l'acide phosphoreux, un thiol tel que le thiophénol ou le méthyl mercaptan, ou encore un thiosulfate, ou un bisulfite, par exemple de sodium. La triphénylphosphine est particulièrement préférée.

On opère au sein d'un solvant organique ou d'un mélange de solvants organiques, de préférence au reflux. On peut citer notamment les solvants aromatiques tels que benzène, toluène, xylène, ou encore le cyclohexane, le tétrahydrofuranne, le dioxanne, le diméthoxyéthane, le monoglyme ou le diglyme, le cas échéant en mélange avec un solvant protique, notamment un alcool tel que le méthanol, l'éthanol ou l'isopropanol.

On opère le cas échéant en présence d'un carbonate alcalin tel que le carbonate de sodium ou le carbonate de calcium.

A titre purement indicatif, on peut ajouter que dans l'application selon l'invention, il est vraisemblable qu'au cours du procédé, la forme allylique du sulfoxyde de formule (V) se trouve en équilibre avec la forme sulfénate (Vₐ) : 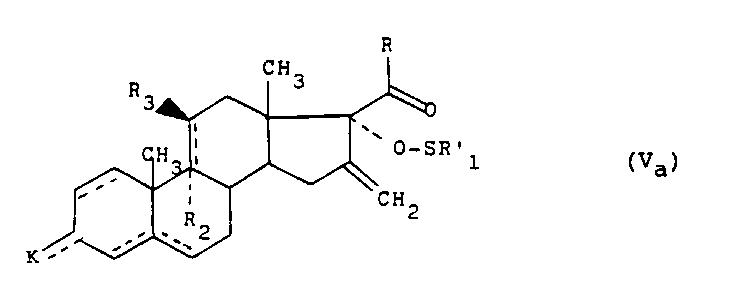 laquelle subit une réduction pour conduire au composé (A) désiré.

L'invention a notamment pour objet une application telle que définie plus haut, caractérisée en ce que l'on soumet un composé de formule (I_{A}) telle que définie plus haut, à l'action d'au moins deux équivalents d'un agent oxydant, pour obtenir un composé de formule (I_{c}) telle que définie plus haut, que l'on soumet, à chaud, à l'action d'un agent thiophile, pour obtenir le composé de formule (A) attendu.

L'invention a aussi notamment pour objet, une application telle que définie plus haut, caractérisée en ce que l'on soumet un composé de formule (I_{c}) telle que définie plus haut, à chaud, à l'action d'un agent thiophile, pour obtenir le composé de formule (A) attendu.

Il est bien entendu possible de déprotéger les produits obtenus comportant un ou deux radicaux hydroxy protégés en utilisant les méthodes connues de l'homme du métier.

L'invention a enfin pour objet, à titre de composés industriels et notamment à titre d'intermédiaires utiles dans l'application selon l'invention, les composés de formule (IV), telle que définie plus haut, ainsi que les composés de formule (V_{b}) : 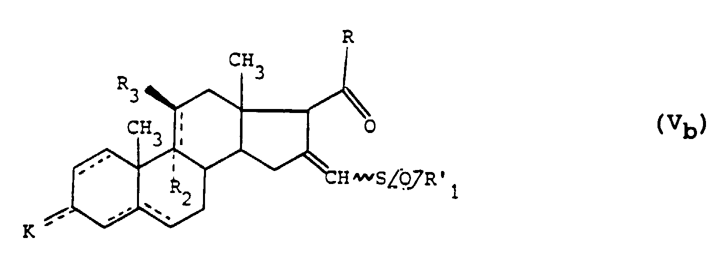 correspondant aux composés de formule (V), telle que définie plus haut, dans laquelle le trait pointillé en position 16(17) et 16(16') représente une seconde liaison uniquement en position 16(16') et les composés de formule (V_{c}) : 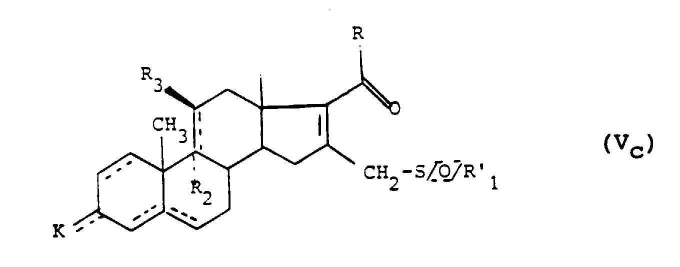 correspondant aux composés de formule (V), telle que définie plus haut, dans laquelle le trait pointillé en position 16(17) et (16(16') représente une seconde liaison uniquement en position 16(17), à l'exception de ceux dans lesquels R représente un radical méthyle.

Les composés de formule (A) dans laquelle R représente un radical méthyle sont décrits notamment dans les brevets américains 3354184, 3178462, 3312692, 3359287, 3064015, 3519619 ou peuvent être obtenus au départ des composés décrits dans ces brevets, par des procédés connus de l'homme du métier. Ceux dans laquelle R représente un radical -CH₂OH libre ou protégé sont quant à eux décrits par exemple dans les brevets allemands 1263765, 1263766, les brevets américains 3350394, 4567001, 3354184, le brevet français 1285336 ou encore les brevets européens 104054 ou 174496, ou peuvent être obtenus par des procédés connus de l'homme du métier au départ des composés décrits dans ces brevets ou encore dans les brevets américains 3309272 ou 3178462.

Les composés de formule (A) sont utiles comme intermédiaires dans la synthèse de composés thérapeutiquement actifs.

Les composés de formule (II) dans laquelle R représente un radical méthyle sont décrits notamment dans les brevets américains 2705719, 2817671, le brevet français 1058850, le brevet belge 711016 ou les brevets anglais 881501, 2199325 ou peuvent être obtenus au départ des composés décrits dans ces brevets, ainsi que dans les brevets allemands 2207420 ou américains 4113722 et 3976638, par des procédés connus de l'homme du métier. Les composés de formule (II) dans laquelle R représente un radical -CH_{**2**}OH libre ou protégé sont décrits par exemple dans les brevets américains 2802839, 2745852, 2773058, 2864834, les brevets belges 540478 ou 789387, le brevet allemand 2207420, néerlandais 6902507 ou soviétique 819119, ou peuvent être obtenus au départ des composés décrits dans ces brevets, ainsi que dans les brevets américains 3976638 ou 2966504, européen 123736 ou canadien 760431, par des procédés connus de l'homme du métier.

Les réactifs de formule (P) dans laquelle R_{**1**} représente un radical méthyle et phényle sont décrits dans J. Chem. Soc. Chem. Comm. 1983, 835, 1978, 362 ou encore J. Org. Chem. 1978, 43, 3101. Les autres réactifs de formule (P) peuvent être préparés par des méthodes analogues à celles décrites dans les références ci-dessus.

Les exemples suivants illustrent l'invention sans toutefois la limiter.

### Exemple 1 : 21-acétoxy 16alpha-[bis(phénylthio) méthyl]-pregna-1,4,9(11)triène-3,20-dione.

### Stade A : 21-acétoxy-16alpha-[(phénylthio)-nitro-méthyl]-1,4,9(11)-pregnatriène 3,20-dione.

2,01 g de 21-acétoxy-1,4,9(11),16-pregnatétraèn-3,20-dione et 1,1 g de (phénylthio)-nitro-méthane sont dissous, sous atmosphère inerte et à l'abri de la lumière, dans un mélange de THF sec (20 ml) et de tertiobutanol (20 ml). On ajoute 0,8 ml de DBU (1,8-diazabicyclo[5,4,0]undec-7-ène) et le mélange réactionnel est agité à température ambiante pendant 8 h. Une solution de 2 g d'acide citrique dans 50 ml d'eau est alors ajoutée. Après extraction au dichlorométhane, filtration sur silice, séchage sur sulfate de sodium, filtration et évaporation du solvant à pression réduite, on obtient 3,73 g de produit brut. L'excès de (phénylthio)-nitro-méthane est éliminé en reprenant le brut de réaction dans un mélange pentane (75 ml)-éther (30 ml). On obtient 2,79 g de 21-acétoxy-16alpha[(phénylthio)-nitro-méthyl]-1,4,9(11)-pregnatrièn-3,20-dione. F=92-100°C.

### Spectre IR (cm⁻¹) :

1740(s) ; 1720(s) ; 1655(s) ; 1615 (w) ; 1595 (w) ; 1540(s) (NO_{**2**}) ; 885 (w) (C-SPh)

### Spectre RMN ¹H :

2 isomères visibles : 0,72 et 0,73 (3H,s,Me-18) ; 1,41 (3H,s,Me-19) ; 2,17 et 2,20 (3H,s,COCH_{**3**}) ; 2,67 et 2,97 (1H,d,J=9,2Hz,C_{**17**}-H) ; 3,60 (1H,m,W_{**1/2**}=35Hz, C_{**16**}-H) ; 4,43 et 4,75 (2H,système AB,J=16,9Hz) et 4,58 et 4,88 (2H,système AB,J=17,0 Hz) ; 5,38 (1H,d,J=8,14 Hz,C_{**16**}'-H) et 5,43 (1H,d,J=5,9 Hz,C_{**16**}'-H) ; 5,55 (1H,massif, w_{**1/2**}=10 Hz,C_{**11**}-H) ; 6,08 (1H,massif,W1/2=10 Hz, C_{**4**}-H) ; 6,30 (1H,d-d,J=10,2 Hz, J=1,7 Hz, C_{**2**}-H) ; 7,17 (1H,d,J=10,2 Hz, C_{**1**}-H), 7,34-7,48 (5H,massif).

### Spectre RMN ¹³C :

30 carbones, 2 isomères :
pairs : 29,87 et 30,65 (s) ; 32,00(s) ; 34,60 (s) ; 44,38 et 44,60(q) ; 45,91(q) ; 69,01(s) ; 130,58(q) ; 130,98(q) ; 143,21 et 143,34 (q) ; 166,03 (q) ; 170,40(q) ; 186,24 (q) ; 201,40 et 201,65(q).
impairs : 13,64(p, 18-Me) ; 20,52(p,COCH_{**3**}) ; 26,61(p, 19-Me) ; 36,29(t) ; 40,05 et 40,17 (t) ; 40,97 (t) ; 52,21 et 52,62(t) ; 61,95(t) ; 98,03 et 98,54(t) ; 119,65(t) ; 124,18(t) ; 127,62(t) ; 129,71(t) ; 129,87(t) ; 132,94(t) ; 133,23(t) ; 154,17(t).

### Spectre de masse :

m/z=512(M+) ; 483 ; 391 ; 314 ; 213.

### Stade B : 21-acétoxy-16alpha-[bis(phénylthio)-méthyl] 1,4,9(11)-pregnatriène 3,20-dione.

2 ml de thiophénol (5 ml) et d'acide acétique sont mélangés et portés à ébullition sous azote. 2,51 g de 21-acétoxy-16alpha-[(phénylthio)-nitro-méthyl]-1,4,9(11)-pregnatrièn-3,20-dione sont additionnés et le mélange est laissé à reflux pendant 4 h 30 mn. Une fois le mélange refroidi, de l'éther est ajouté (25 ml). Après neutralisation de l'acide acétique par une solution saturée d'hydrogénocarbonate de sodium, on ajoute 25 ml d'éther. La phase organique est récupérée, lavée à l'eau, puis séchée et concentrée. On obtient 4 g d'une huile marron. L'élimination du thiophénol en excès se fait en reprenant le brut de réaction dans un mélange éther (25 ml)-pentane (100 ml). On obtient 1,70 g du dérivé disulfure attendu, F=98-100°C après purification par chromatographie sur silice (éluant : éther-dichlorométhane 9-1). [alpha]_{**D**}= -13,4° (C=12,7 mg/ml ; chloroforme).

### Spectre IR (cm⁻¹) :

1750(s) ; 1720(s) ; 1665(s) ; 1630(m) ; 1610(w) ; 1585(w) ; 1550(w) ; 1480(w) ; 1440(w) ; 1410(w) ; 1370(w) ; 1270(m) ; 1120(w) ; 1155(w) ; 1070(m) ; 1050(m) ; 1025(m) ; 890(m).

### Spectre RMN ¹H :

0,61(3H,s,Me-18) ; 1,37(3H,s,Me-19) ; 2,10(3H,s,COCH_{**3**}) ; 2,87(1H,d,J=9,2HZ,C_{**17**}-H); 3,31(1H,m,W_{**1/2**}=19 Hz,C_{**16**}-H) ; 4,25 et 4,68(2H,système AB,J=16,9 Hz) ; 4,33(1H,d,J=4,8 Hz,C_{**16**}'-H) ; 5,47(1H,massif,W_{**1/2**}=9Hz,C_{**11**}-H) ; 6,03(1H,massif,W_{**1/2**}=5 Hz,C_{**4**}-H) ; 6,23(1H,d-d,J=10,1 Hz,J=1,7 Hz,C_{**2**}-H) ; 7,10(1H,d,J=10,2 Hz,C_{**1**}-H), 7,18-7,41(10H,massif).

### Spectre RMN ¹³C :

pairs : 30,86(s) ; 32,14 (s) ; 34,64 (s) ; 40,23 (s) ; 44,54 (q) ; 134,40(q) ; 143,38(q) ; 154,45(q) ; 166,50(q) ; 170,32(q) ; 186,31(q) ;202,50(q). impairs : 13,81(p) ; 20,53(p) ; 26,61(p) ; 36,50(t) ; 43,52(t) ; 45,98(t) ; 52,75(t) ; 62,47(t) ; 64,64(t) ; 69,09(t) 119,89(t) ; 124,03(t) ; 127,49(t) ; 127,88(t) ; 127,97(t) ; 129,16(t) ; 132,27(t) ; 132,57(t).

### Spectre de masse :

m/z=489(M+ - Sph).

### Exemple 2 : 21 -acétoxy-16alpha-[(phénylthio)(phénylsulfinyl) méthyl]-1,4,9(11)-pregnatriène 3,20-dione.

On dissout 60 mg de disulfure obtenu à l'exemple 1 dans 1 ml de dichlorométhane et on refroidit à -60°C. On ajoute 26 mg d'acide méta-chloroperbenzoïque. Après une heure, on rajoute 5 mg d'acide métachloroperbenzoïque. Le mélange réactionnel est laissé encore à -60°C pendant 1 heure. On ajoute 2 ml d'une solution saturée d'hydrogénocarbonate de sodium ; ramène le mélange à température ambiante et extrait au dichlorométhane (2x1,5 ml). La phase organique est ensuite lavée à l'eau (3x2 ml), puis séchée et le solvant est évaporé sous pression réduite. On obtient 56 mg de monosulfoxyde brut attendu.

Ce dérivé est instable et est conservé au réfrigérateur.

### Exemple 3 : 21-acétoxy-16alpha-[bis(phénylsulfinyl)-méthyl]-1,4,9(11)-pregnatriène-3,20-dione.

On dissout 1,09 g de produit obtenu à l'exemple 1 dans 10 ml de dichlorométhane et refroidit à -78°C. On ajoute 796 mg d'acide méta-chloroperbenzoïque à 80 % et maintient le mélange réactionnel à -78°C pendant 3 heures. On ajoute 15 ml d'une solution saturée d'hydrogénocarbonate de sodium ; le mélange est ramené aussitôt à température ambiante et extrait au dichlorométhane. La phase organique est ensuite lavée à l'eau, puis séchée et le solvant est évaporé sous pression réduite. Le disulfoxyde obtenu est purifié par chromatographie sur silice (éluant : cyclohexane : acétate d'éthyle 1/1) ; on obtient 649 mg du composé attendu.

### Spectre IR (cm⁻¹) :

3050, 2960, 2920, 1745(s), 1720(s), 1660(s), 1625, 1615(m), 1580, 1450(m), 1375, 1320, 1270, 1240(s), 1150(m), 1090(m), 1050(m), 890.

### Spectre RMN ¹H :

1er isomère : 0,62(3H,s,Me-18) ; 1,40(3H,s,Me-19) ; 2,18(3H,s,COCH_{**3**}) ; 3,43-3,54(3H,16-H,16'-H et 17-H) ; 4,29 et 4,59(2H,système AB,J=16,6 Hz) ; 5,51(1H,massif,W_{**1/2**}=10 Hz,C_{**11**}-H) ; 6,08(1H,massif,W1/2=5 Hz,C_{**4**}-H) ; 6,28(1H,d-d,J=10,2 Hz,J=1,7 Hz,C_{**2**}-H) ; 7,15(1H,d,J=10,1 Hz,C_{**1**}-H) , 7,31-7,66(10H,massif).
2ème isomère : 0,68(3H,s,Me-18) ; 1,40(3H,s,Me-19) ; 2,13(3H,s,COCH_{**3**}) ; 3,54-3,62(3H,16H,16'-H et 17-H) ; 4,44 et 4**,**79 (2H,système AB,J=16,5 Hz) ; 5,58(1H,massif,W_{**1/2**}=10 Hz,C_{**11**}-H) ; 6,08(1H,massif,W1/2=5 Hz,C_{**4**}-H) ; 6,28(1H,dd,J=10,2 Hz,J=1,7 Hz,C_{**2**}-H) ; 7,18(1H,d,J=10,1 Hz,C_{**1**}-H), 7,31-7,66(10H,massif).

### Exemple 3' : 21-acétoxy 16alpha-[(phénylthio) (phénylsulfinyl)] et 16alpha-[bis(phénylsulfinyl)-méthyl]-1,4,9(11)-pregnatriène-3,20-diones.

0,206 g de disulfure obtenu à l'exemple 1 est dilué dans 2 ml d'acide acétique, sous argon. On refroidit à 0°C par un bain de glace. On ajoute au mélange 5 gouttes d'eau oxygénée 30 %. On additionne quelques gouttes de dichlorométhane, laisse remonter la température. Après 21 heures à température ambiante, on obtient majoritairement le monosulfoxyde identifié comparativement au produit de l'exemple 2. On poursuit la réaction pendant 50 heures environ sous atmosphère inerte et à température ambiante, puis l'arrête par addition d'eau.

On obtient, après traitement comme à l'exemple 3, le disulfoxyde attendu, identique à celui obtenu à l'exemple 3.

### Exemple 4 : 21-acétoxy 16-méthylène 17alpha-hydroxy pregna 1,4,9(11)triène-3,20-dione.

### Stade A : 21-acétoxy 16alpha-[(phénylthio) méthyl] 1,4,9(11) 16-pregnatétraène 3,20-dione et dérivé 16alpha-[(phénylthio) méthylène] correspondant.

On reprend le monosulfoxyde obtenu comme à l'exemple 2, à partir de 204 mg de disulfure, dans 3 ml de toluène sec. On ajoute 101 mg de triphénylphosphine et la solution est chauffée à reflux sous argon pendant 15 heures. Après 6 heures de chauffage, on ajoute 55 mg de triphénylphosphine. Le toluène est ensuite évaporé et le produit est purifié par chromatographie sur silice (éluant : cyclohexane/acétate d'éthyle 9/1 puis 3/1). On obtient 143 mg d'un mélange de deux isomères de position pour la double liaison en position 16 (delta16-16' ou delta16-17).

### Spectre IR (cm⁻¹) :

3020, 2910, 2820, 1750(s), 1720(m), 1660(s), 1620(m), 1580, 1550, 1430, 1370, 1260, 1040, 880.

### Spectre RMN ¹H :

0,80(3H,s,Me-18) ; 1,41 et 1,42(3H,s,Me-19) ; 2,18 et 2,19(3H,s,COCH_{**3**}) ; 3,38(1H,m,W_{**1/2**}=10 Hz,C_{**17**}-H) ; 3,72 et 4,09(2H,système AB,J=13,6 Hz,C_{**16**}'-H) ; 4,44 et 4,67(2H,système AB,J=16,6 Hz) et 4,71 et 4,90(2H,système AB,J=17 Hz) ; 5,50 et 5,59(1H,massif,W_{**1/2**}=12,5 Hz,C_{**11**}-H) ; 6,09(1H,massif,W1/2=5 Hz,C_{**4**}-H) ; 6,30(1H,d-d,J=10,2 Hz,J=1,7 Hz,C_{**2**}-H) ; 6,31 et 6,27(1H,d,J=1,9 Hz,C_{**16**}'-H), 7,16-7,52(6H,massif).

### Spectre RMN ¹³C :

pairs : 32,03 ; 34,27 ; 34,66 ; 36,66 ; 37,12 ; 37,77 ; 40,41 ; 44,05 ; 45,97 ; 47,09 ; 68,08 ; 69,01 ; 134,58 ; 136,18 ; 143,06 ; 143,79 ; 144,11 ; 145,90 ; 151,58 ; 166,37 ; 170,29 ; 186,34 ; 193,57 ; 200,77.

impairs : 14,16 ; 16,02 ; 20,53 ; 26,54 ; 28,72 ; 35,73 ; 51,19 ; 63,20 ; 117,37 ; 119,84 ; 120,23 ; 123,97 ; 126,43 ; 127,43 ; 128,54 ; 129,02 ; 129,09 ; 131,72 ; 154,47.

### Spectre de masse :

m/z=490, 489, 488(M+), 448, 447, 446(M+-COCH_{**3**}), 430, 415, 387, 319, 207.

### Stade B : 21-acétoxy 16alpha-[(phénylsulfinyl) méthyl] 1,4,9(11) 16-pregnatétraène-3,20-dione et dérivé 16alpha-[(phénylsulfinyl) méthylène] correspondant.

On dissout 6,42 g du mélange d'isomères obtenu comme décrit au stade précédent, dans 100 cm^{**3**} de dichlorométhane et refroidit à -78°C sous azote. On ajoute 2,92 g d'acide métachloroperbenzoïque à 80 % et maintient sous agitation pendant 2 heures environ.

On traite à basse température par une solution saturée de bicarbonate de sodium puis à l'eau, sèche et évapore à sec. On obtient après chromatographie sur silice (élution : acétate d'éthyle puis acétate d'éthyle/méthanol 9/1) : 3,44 g du mélange de sulfoxydes désiré.

### Stade c : 21-acétoxy 16-méthylène 17alpha-hydroxy pregna 1,4,9(11)-triène-3,20-dione.

On dissout le produit obtenu au stade précédent dans un mélange de 50 cm^{**3**} de toluène et 50 cm^{**3**} de méthanol et ajoute 2,68 g de triphénylphosphine. On chauffe à 85-90°C pendant 20 heures, puis refroidit et évapore le solvant.

On obtient après chromatographie sur silice (élution : éther/dichlorométhane/éther de pétrole 4/1/5), 1,97 g de produit attendu. F=213°-215°C (dec) après cristallisation dans le méthanol.
[alpha]_{**D**}= -32,8° (C=10,5 mg/ml ; chloroforme).

### Spectre IR (cm⁻¹) :

3200 ; 2900 ; 2850 ; 2250 ; 1750(s) ; 1730(s) ; 1675(s) ; 1625 ; 1605 ; 1440 ; 1410 ; 1370 ; 1235(s) ; 1075 ; 915(s).

### Spectre RMN ¹H :

0,70(3H,s,Me-18) ; 1,41(3H,s,Me-19) ; 2,17(3H,s,COCH_{**3**}) ; 4,93 et 5,07(2H,système AB,J=17,6 Hz) ; 5,02(1H,s,c=CH_{**2**}) ; 5,15(1H,s,c=CH_{**2**}) ; 5,60(1H,massif,W_{**1/2**}=12 Hz,C_{**11**}-H) ; 6,07(1H,massif,W1/2=5 Hz,C_{**4**}-H) ; 6,29(1H,d-d,J=10,2 Hz,J=1,4 Hz,C_{**2**}-H) ; 7,20(1H,d,J=10,2 Hz,C_{**1**}-H).

### Spectre RMN ¹³C :

pairs : 31,96(s) ; 32,25(s) ; 33,36(s) ; 35,05(s) ; 46,04(q) ; 45,45(q) ; 68,65(s) ; 89,65(q) ; 114,45(s) ; 142,28(q) ; 152,64(q) ; 166,49(q) ; 170,64(q) ; 186,37(q) ; 204,00(q).

impairs : 14,18(p) ; 20,60(p) ; 26,81(p) ; 36,38(t) ; 46,31(t) ; 120,83(t) ; 124,05(t) ; 127,50(t) ; 154,51(t).

### Exemple 5 : 21-acétoxy 16-méthylène 17alpha-hydroxy pregna 1,4,9(11)-triène-3,20-dione.

On dissout 205 mg du mélange obtenu comme décrit au stade A de l'exemple 4 dans 3 ml de dichlorométhane et refroidit à -78°C sous azote. On ajoute 91 mg d'acide méta-chloroperbenzoïque à 80 %. Après 2 heures de réaction, le produit de départ a complètement disparu. On ajoute 221 mg de triphényl phosphine à basse température, puis on évapore le dichlorométhane sous pression réduite et le remplace par 3 ml de toluène sec. Après 2 h 30 mn de chauffage à reflux, on évapore à sec et obtient ainsi après purification par chromatographie sur silice éluant : cyclohexane/éther, 4/1 à 0/1), 113 mg de l'alcool attendu, identique à celui obtenu à l'exemple 4 F=213-215°C.

### Exemple 6 : 21-acétoxy 16-méthylène 17alpha-hydroxy pregna 1,4,9(11)-triène-3,20-dione.

On dissout 648 mg de disulfoxyde obtenu à l'exemple 3 sous argon, dans 10 cm^{**3**} de toluène et la solution est refroidie à -60°C. On ajoute 500 mg de carbonate de calcium et le mélange réactionnel est chauffé à 80°C pendant 2 heures. On ajoute ensuite 540 mg de triphénylphosphine ainsi que 10 cm^{**3**} de méthanol. La solution obtenue est portée à reflux, à 80°C pendant 11 heures, puis refroidie et amenée à sec. Après chromatographie sur silice éluant cyclohexane/acétate d'éthyle ; 7/3 puis 1/1), on récupère 217 mg de l'alcool attendu, identique à celui obtenu à l'exemple 4 F=213-215°C.

### Exemple 6' : 21-acétoxy 16-méthylène 17alpha-hydroxy pregna 1,4,9(11)-triène-3,20-dione.

On dissout 287 mg de disulfoxyde obtenu à l'exemple 3 ou 3' dans 5 ml de toluène sec et on ajoute 250 mg de carbonate de calcium. La solution est chauffée à reflux pendant 75 mn. On ajoute 5 gouttes (1 équivalent) de triméthylphosphite et 5 ml de méthanol, puis 8 gouttes de triméthylphosphine après 8 heures et après 24 heures. Après 34 heures de chauffage à reflux, on observe en chromatographie sur couche mince que la réaction est complète. On laisse refroidir la solution et on additionne alors 15 ml de dichlorométhane et 15 ml d'eau. Le mélange obtenu est filtré et les phases organique et aqueuse sont séparées. La phase organique obtenue est lavée par une solution saturée de bicarbonate et une solution saturée de chlorure de sodium. Après séchage et élimination du solvant, on obtient 256 mg de produit attendu brut que l'on chromatographie sur silice (élution : éther de pétrole/acétate d'éthyle 7/3 puis 1/1). On obtient 64 mg soit 36 % de produit attendu pur identique à celui obtenu à l'exemple 4. F=213-215°C.

### Exemple 7 : 21-acétoxy 16-méthylène 17alpha-hydroxy pregna 1,4,9(11)triène-3,20-dione.

On dissout 702 mg de produit obtenu à l'exemple 1, sous azote, dans 10,5 cm^{**3**} de dichlorométhane et la solution est refroidie à -60°C. On ajoute 319 mg d'acide méta-chloroperbenzoïque à 80 %. Après 30 mn, on rajoute 243,6 mg d'acide méta-chloroperbenzoïque et la réaction est laissée entre -60°C et -40°C pendant 5 heures. On ajoute alors 942 mg de triphénylphosphine, évapore le dichlorométhane sous pression réduite et le remplace par 11 cm^{**3**} de toluène sec. La solution obtenue est portée à reflux pendant 7 heures. On refroidit, évapore le solvant et chromatographie le résidu sur colonne de silice éluant cyclohexane/acétate d'éthyle ; 1/0 à 1/1. On récupère 115 mg d'alcool attendu, identique à celui obtenu à l'exemple 4. F=213-215°C.

### Exemple 8 : 16-méthylène 17alpha-hydroxy 4-pregnèn-3,20-dione.

### Stade A : 16alpha-[(phénylthiol-nitro méthyl] 4-pregnèn-3,20-dione.

138 mg de 4,16-pregnadièn-3,20-dione et 110 mg de (phénylthio)-nitro-méthane sont dissous, sous atmosphère inerte et à l'abri de la lumière, dans 2 ml d'un mélange (1-1) de tétrahydrofuranne et de tertiobutanol puis on ajoute 0,1 ml de DBU (1,8-diazabicyclo[5,4,0]undec-7-ène) et le mélange réactionnel est agité à température ambiante pendant 48 h. Une solution de 0,5 g d'acide citrique dans 5 ml d'eau est alors ajoutée. Après extraction au dichlorométhane, séchage sur sulfate de sodium, filtration et évaporation du solvant sous pression réduite, on chromatographie le résidu sur silice en éluant à l'éther et obtient 48,3 mg de produit attendu sous forme d'un mélange 2-1 d'épimères. F = 166-172°C.

### Spectre IR (CHBr₃) :

1702, 1660, 1551 cm^{**-1**}

### Spectre RMN (200MHz) :

^{**1**}H : 7,4 (5H,m) ; 5,7 (lH,s) ; 5,4 (lH,2d) ; 3,55 (lH,m) ; 2,87 (1/3H,d, J=8,8Hz) ; 2,64 (2/3H,d, J=8,8Hz) ; 2,23 (2/3 3H,s) ; 2,16 (1/3 3H,s) ; 1,18 (3H,s) ; 0,71 (3H,s).
^{**13**}C (ppm) :
14,38 ; 17,47 ; 21,04 ; 28,72 ; 29,23 ; 31,58 ; 31,76 ; 32,64; 34,00 ; 35,39 ; 35,83 ; 38,66 ; 38,76 ; 40,67 ; 45,23 ; 53,50; 54,75 ; 55,24 ; 66,86 ; 98,66 ; 98,99 ; 124,26 ; 129,53 ; 129,80 ; 131,45 ; 132,94 ; 170,18 ; 199,19 ; 206,37.

### Stade B : 16-méthylène 17alpha-hydroxy 4-pregnèn-3,20-dione.

En opérant comme indiqué à l'exemple 3 à partir du produit obtenu au stade A ci-dessus, on obtient le disulfoxyde correspondant que l'on transforme comme indiqué à l'exemple 6 pour obtenir la 16-méthylène 17alpha-hydroxy 4-pregnèn-3,20-dione attendue (obtenue comme indiqué dans le brevet US 3,354,184).

### Exemple 9 : 3bêta-acétoxy 16-méthylène 17alpha-hydroxy 5-pregnèn-3,20-dione.

### Stade A : 3bêta-acétoxy-16alpha-[(phénylthio) nitrométhyl] 5-pregnèn-20-one.

124,3 g de 3bêta-acétoxy 5,16-pregnadièn-20-one et 89,7 mg de (phénylthio)-nitro-méthane sont dissous, sous atmosphère inerte et à l'abri de la lumière, dans 2 ml d'un mélange (1-1) de tétrahydrofuranne et de tertiobutanol puis on ajoute 0,05 ml de DBU (1,8-diazabicyclo[5,4,0]undec-7-ène) et le mélange réactionnel est agité à température ambiante pendant 48 h. Une solution de 0,5 g d'acide citrique dans 5 ml d'eau est alors ajoutée. Après extraction au dichlorométhane, séchage sur sulfate de sodium, filtration et évaporation du solvant sous pression réduite, on chromatographie le résidu sur silice en éluant avec un mélange éther-éther de pétrole (1-3) et obtient 66 mg de produit attendu sous forme d'un mélange 2-1 d'épimères. F = 135-159°C.

### Spectre IR (CHBr₃) :

1720, 1702, 1551 cm^{**-1**}

### Spectre RMN (200MHz) :

^{**1**}H : (Δ) 7,4 (5H,m) ; 5,35 (1H,2d) ; 4,6 (1H,m) ; 3,55 (1H,m); 2,85 (1/3H,d, J=8,8Hz) ; 2,64 (2/3H,d, J=8,8Hz) ; 2,23 (2/3 3H,s) ; 2,16 (1/3 3H,s) ; 2,04 (3H,s) ; 1,02 (3H,s) ; 0,66 (3H,s).
^{**13**}C (ppm) :
114,19 ; 19,32 ; 20,88 ; 21,46 ; 27,72 ; 29,36 ; 31,53 ; 36,58; 36,96; 38,04 ; 38,72 ; 40,46 ; 45,19 ; 49,64 ; 55,34 ; 55,83 ; 66,90 ; 73,74 ; 98,73 ; 99,06 ; 121,96 ; 129,43 ; 129,71 ; 132,88 ; 139,72 ; 170,56 ; 206,64.

### Stade B : 3bêta-acétoxy 16-méthylène 17alpha-hydroxy 5-pregnèn-3,20-dione.

En opérant comme indiqué à l'exemple 3 à partir du produit obtenu au stade A ci-dessus, on obtient le disulfoxyde correspondant que l'on transforme comme indiqué à l'exemple 6 pour obtenir la 3bêta-acétoxy 16-méthylène 17alpha-hydroxy 5-pregnèn-20-one (décrit dans le brevet US 3,519,619).

### Exemple 10 : 16-méthylène 17alpha-hydroxy 4-pregna 4,9(11) dièn-3,20-dione.

### Stade A : 16alpha-[(phénylthio) nitrométhyl] 4,9(11)-pregnadièn-3,20-dione.

100 mg de pregna 4,9(11),16-trièn-3,20-dione et 100 mg de (phénylthio)-nitro-méthane sont dissous, sous atmosphère inerte et à l'abri de la lumière, dans 1 ml d'un mélange (1-1) de tétrahydrofuranne et de tertiobutanol puis on ajoute 30 mg de DBU (1,8-diazabicyclo[5,4,0]undec-7-ène) et le mélange réactionnel est agité à température ambiante pendant 16 h. Une solution de 0,5 g d'acide citrique dans 5 ml d'eau est alors ajoutée. Après extraction au dichlorométhane, séchage sur sulfate de sodium, filtration et évaporation du solvant sous pression réduite, on chromatographie le résidu sur silice en éluant avec un mélange éther de pétrole-acétate d'éthyle (2-1) et obtient 129 mg de produit attendu sous forme d'un mélange 2-1 d'épimères. F = 198-200°C (déc.) après recristallisation dans un mélange chloroforme-méthanol.

### Spectre IR (CHBr₃) :

1707, 1665, 1614, 1552 cm^{**-1**}

### Spectre RMN (200mHz) :

^{**1**}H : (Δ) 7,4 (5H,m) ; 5,75 (1H,s) ; 5,55 (1H,m) ; 5,4 (1H,2d); 3,6 (1H,m); 2,95 (1/3H,d, J=9Hz) ; 2,75 (2/3H,d, J=9Hz) ; 2,24 (2/3 3H,s) ; 2,17 (1/3 3H,s) ; 1,34 (3H,s) ; 0,65 (3H,s).
^{**13**}C (ppm) :
13,96 ; 26,16 ; 29,50 ; 30,08 ; 31,28 ; 31,98 ; 32,60 ; 33,90; 34,25 ; 36,94 ; 40,70 ; 41,07 ; 43,30 ; 43,47 ; 51,58 ; 52,09; 66,50 ; 66,68 ; 98,46 ; 98,81 ; 117,84 ; 124,26 ; 129,41 ; 129,70 ; 132,81 ; 145,10 ; 198,70 ; 206,03.

### Stade B : 16-méthylène 17alpha-hydroxy pregna 4,9(11) dièn-3,20-dione.

En opérant comme indiqué à l'exemple 3 à partir du produit obtenu au stade A ci-dessus, on obtient le disulfoxyde correspondant que l'on transforme comme indiqué à l'exemple 6 pour obtenir la 16-méthylène 17alpha-hydroxy pregna 4,9(11)-dièn-3,20-dione attendue (décrite dans le brevet US 3,359,287).

## Revendications

1. Les composés de formule (I) : 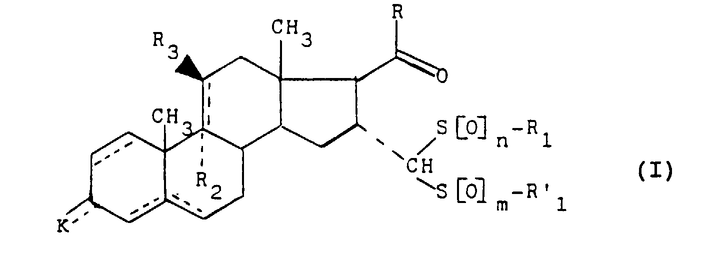 dans laquelle 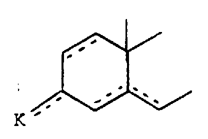 représente soit un système 3-céto Δ4 soit un système 3-céto Δ1,4 soit un système 3-OR₄ Δ5 dans lequel R₄ représente un atome d'hydrogène ou un groupement protecteur du radical hydroxy, R représente un radical méthyle, un radical -CH₂OH ou un radical -CH₂-OR', dans lequel R' représente un groupement protecteur du radical hydroxy, R₁ et R'₁, identiques ou différents, représentent un radical méthyle, un radical alkyle ramifié ne possédant pas d'atome d'hydrogène en position béta, renfermant de 5 à 8 atomes de carbone, un radical aryle renfermant jusqu'à 10 atomes de carbone, un radical hétéroaryle renfermant jusqu'à 10 atomes de carbone et un ou plusieurs hétéroatomes choisis parmi l'azote, le soufre et l'oxygène, ou un radical benzyle, n et m, identiques ou différents, représentent les nombres 0 ou 1, R₂ et R₃ représentent un atome d'hydrogène ou R₂ représente un atome de fluor et R₃ représente un radical formyloxy ou acétyloxy et les traits pointillés en position 9(11) symbolisent la présence éventuelle d'une seconde liaison.

2. Les composés de formule (I), telle que définie à la revendication 1, dans laquelle R représente un radical méthyle, un radical -CH₂OH ou un radical -CH₂OR" dans lequel R" représente un radical alkyle renfermant de 1 à 4 atomes de carbone, un radical acyle renfermant de 1 à 5 atomes de carbone ou un radical benzyle, R₁ et R'₁, identiques ou différents, représentent un radical méthyle, phényle, tolyle ou benzyle, n, m, R₂, R₃ et les traits pointillés étant définis comme à la revendication 1.

3. Les composés de formule (I) tels que définis à la revendication 1 ou 2, répondant à la formule (I_{A}) : 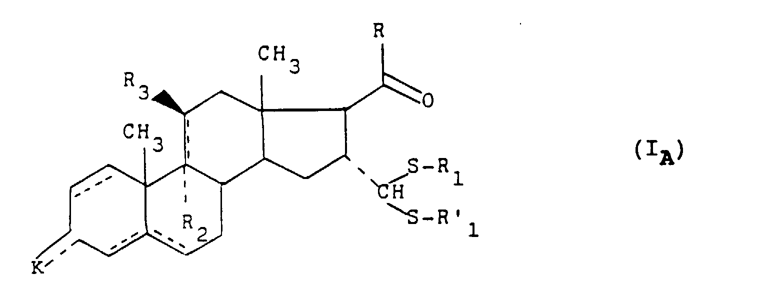 dans laquelle K, R, R₁, R'₁, R₂, R₃ et les traits pointillés sont définis comme précédemment,

4. Les composés de formule (I), tels que définis à la revendication 1 ou 2 répondant à la formule (I_{B}) : 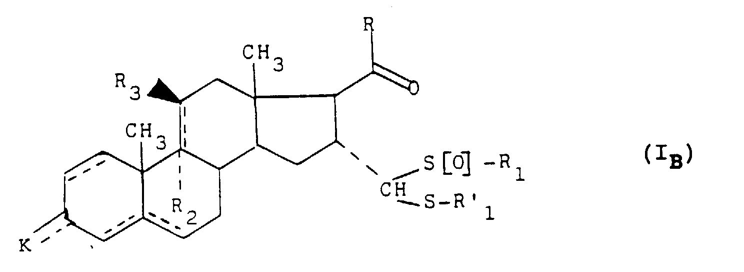 dans laquelle K, R, R₁, R'₁, R₂, R₃ et les traits pointillés sont définis comme précédemment ;

5. Les composés de formule (I), tels que définis à la revendication 1 ou 2, répondant à la formule (I_{c}) : 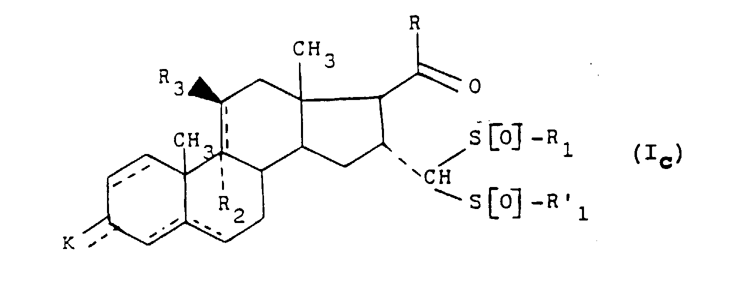 dans laquelle K, R, R₁, R'₁, R₂, R₃ et les traits pointillés sont définis comme à la revendication 1 ou 2.

6. Les composés de formule (I), tels que définis à l'une quelconque des revendications 1 à 5, caractérisés en ce que 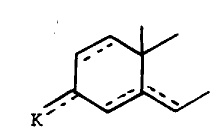 représente un système 3-céto Δ1,4 et R représente un radical -CH₂-OR", R" étant défini comme à la revendication 2, R₃ représente un atome d'hydrogène et les traits pointillés en position 9(11), représentent une seconde liaison.

7. Procédé de préparation des composés de formule (I), telle que définie à la revendication 1, caractérisé en ce que l'on soumet un composé de formule (II) : 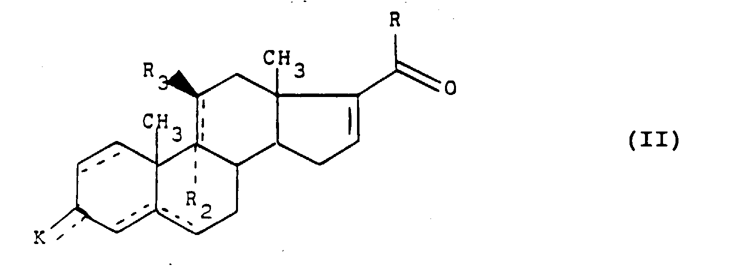 dans laquelle K, R, R₂, R₃ et les traits pointillés ont la signification indiquée plus haut, en milieu basique, à l'action d'un réactif de formule (P) :
R₁S-CH₂-NO₂ (P)
dans laquelle R₁ a la signification indiquée plus haut, pour obtenir un composé de formule (III) : 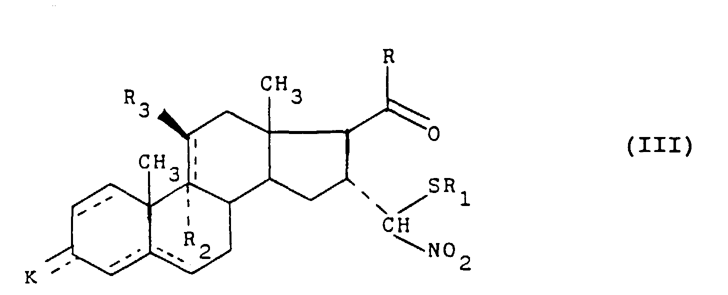 dans laquelle K, R, R₁, R₂, R₃ et les traits pointillés ont la signification indiquée plus haut, que l'on soumet, en présence d'un acide, à l'action d'un thiol ou d'un thiophénol de formule :
HS-R'₁
dans laquelle R'₁ a la signification précitée, pour obtenir un composé de formule (I_{A}) : 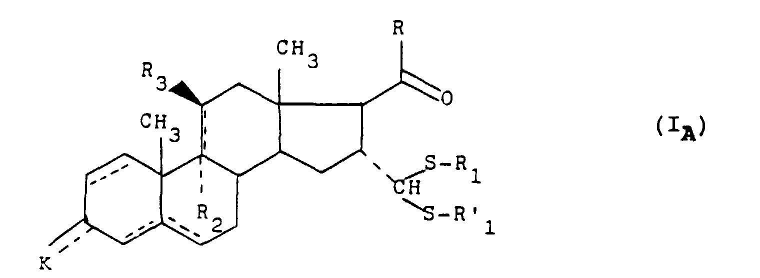 dans laquelle K, R, R₁, R'₁, R₂, R₃ et les traits pointillés ont la signification précitée, correspondant à un composé de formule (I) dans laquelle m et n sont égaux à 0, composé de formule (I_{A}) que, le cas échéant, l'on soumet soit à l'action d'un équivalent d'un agent oxydant, pour obtenir un composé de formule (I_{B}) : 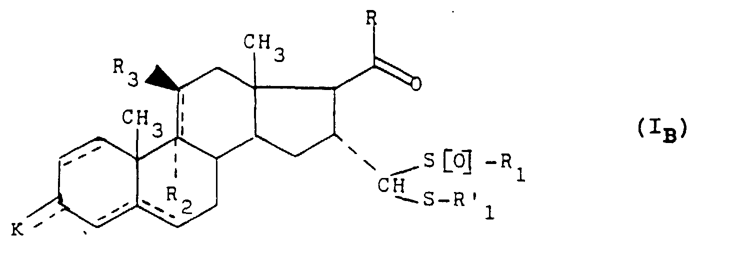 dans laquelle K, R, R₁, R'₁, R₂, R₃ et les traits pointillés ont la signification précitée, correspondant à un composé de formule (I) dans laquelle l'une des valeurs m et n est égale à I et l'autre à 0, soit à l'action d'au moins deux équivalents d'un agent oxydant, pour obtenir un composé de formule (I_{c}) : 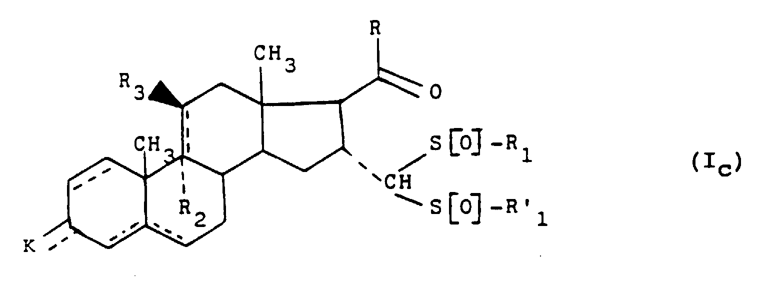 dans laquelle K, R, R₁, R'₁, R₂, R₃ et les traits pointillés ont la signification précitée, correspondant à un composé de formule (I) dans laquelle m et n sont égaux à 1.

8. Procédé selon la revendication 10, caractérisé en ce que l'on soumet un composé de formule (II) : 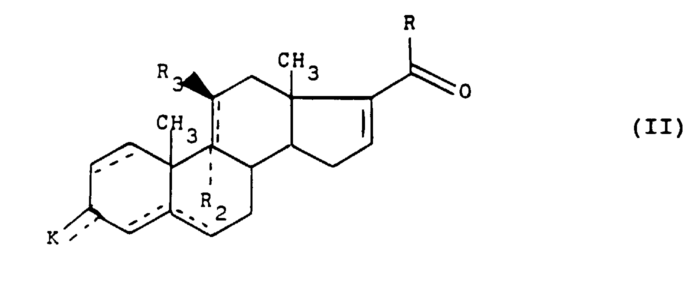 dans laquelle K, R, R₂, R₃ et les traits pointillés ont la signification indiquée plus haut, en milieu basique, à l'action d'un réactif de formule (P) :
R₁S-CH₂NO₂ (P)
dans laquelle R₁ a la signification indiquée plus haut, pour obtenir un composé de formule (III) : 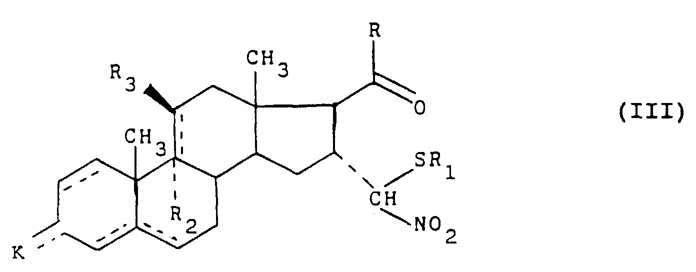 dans laquelle K, R, R₁, R₂, R₃ et les traits pointillés ont la signification indiquée plus haut, que l'on soumet en présence d'un acide à l'action d'un thiol ou d'un thiophénol de formule :
HS-R'₁
dans laquelle R'₁ a la signification précitée, pour obtenir un composé de formule (I_{A}) : 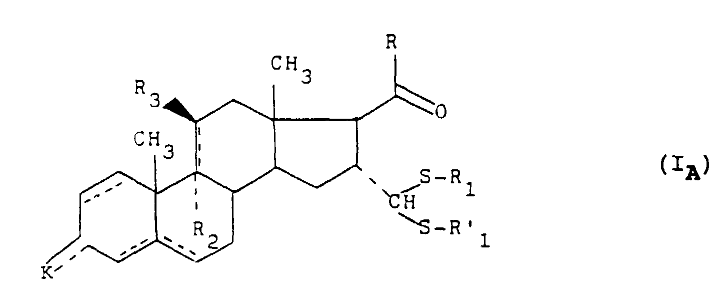 dans laquelle K, R, R₁, R'₁, R₂, R₃ et les traits pointillés ont la signification précitée, correspondant à un composé de formule (I) dans laquelle m et n sont égaux à 0, composé de formule (I_{A}) que l'on soumet à l'action d'au moins deux équivalents d'un agent oxydant, pour obtenir un composé de formule (I_{c}) : 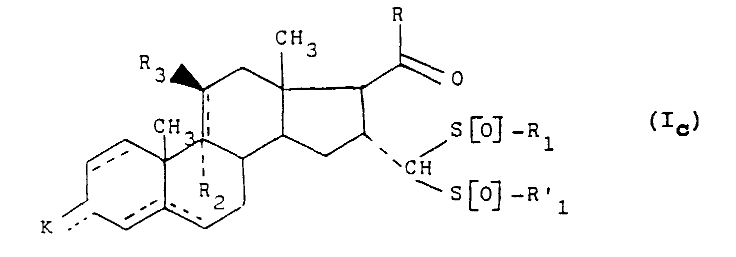 dans laquelle K, R, R₁, R'₁, R₂, R₃ et les traits pointillés ont la signification précitée, correspondant à un composé de formule (I) dans laquelle m et n sont égaux à 1.

9. Procédé selon la revendication 7 ou 8, caractérisé en ce que :
- l'action du réactif de formule (P) sur le composé de formule (II) est effectuée en présence d'une base aminée ;
- l'action du thiol ou du thiophénol est catalysée par un acide carboxylique ;
- l'agent oxydant est un peracide ou l'eau oxygénée en présence d'un acide carboxylique.

10. A titre de composés industriels nouveaux, les composés de formule (III), telle que définie à la revendication 7.

11. Application des composés de formule (I), telle que définie à la revendication 1, à la préparation des composés de formule (A) : 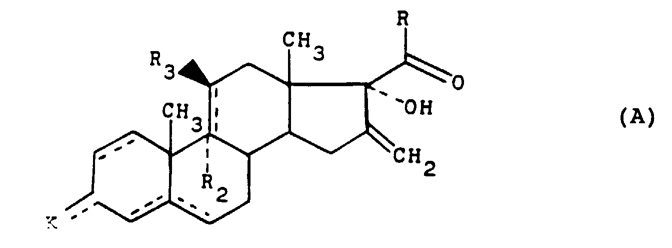 dans laquelle K, R, R₂, R₃ et les traits pointillés ont la signification indiquée à la revendication 1, caractérisée en ce que :
- ou bien l'on soumet un composé de formule (I_{A}) telle que définie à la revendication 3, à l'action d'au moins deux équivalents d'un agent oxydant, pour obtenir un composé de formule (I_{C}) telle que définie à la revendication 5, que l'on soumet, à chaud, à l'action d'un agent thiophile, pour obtenir le composé de formule (A) attendu ;
- ou bien l'on soumet un composé de formule (I_{C}) telle que définie à la revendication 5, à chaud, à l'action d'un agent thiophile, pour obtenir le composé de formule (A) attendu ;
- ou bien l'on chauffe un composé de formule (I_{B}) telle que définie à la revendication 4, le cas échéant en présence d'un agent thiophile, pour obtenir un composé de formule (IV) : 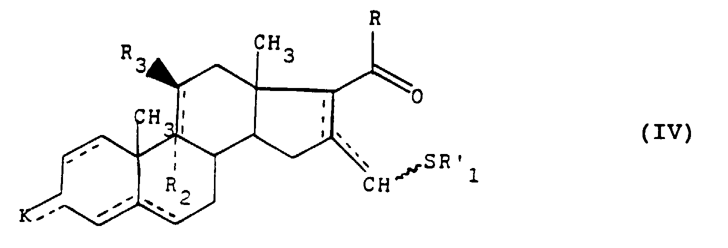 dans laquelle K, R, R'₁, R₂, R₃ et les traits pointillés ont la signification indiquée à la revendication 1, le trait pointillé en 16(17) et 16(16') symbolise l'existence d'un mélange de sulfure vinylique et allylique et le trait ondulé symbolise l'existence d'un mélange d'isomères, composé de formule (IV) que l'on soumet à l'action d'au moins un équivalent d'un agent oxydant, pour obtenir un composé de formule (V) : 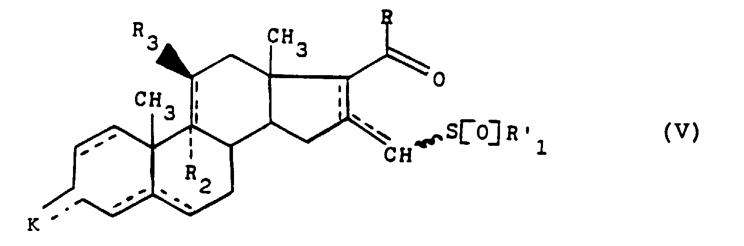 dans laquelle K, R, R'₁, R₂, R₃ les traits pointillés et le trait ondulé ont la signification indiquée ci-dessus, que l'on soumet, à chaud, à l'action d'un agent thiophile, pour obtenir le composé de formule (A) attendu.

12. Application selon la revendication 11, caractérisée en ce que l'on soumet un composé de formule (I_{A}) telle que définie à la revendication 3, à l'action d'au moins deux équivalents d'un agent oxydant, pour obtenir un composé de formule (I_{C}) telle que définie à la revendication 5, que l'on soumet, à chaud, à l'action d'un agent thiophile, pour obtenir le composé de formule (A) attendu.

13. Application selon la revendication 11, caractérisée en ce que l'on soumet un composé de formule (I_{c}) telle que définie à la revendication 5, à chaud, à l'action d'un agent thiophile, pour obtenir le composé de formule (A) attendu.

14. Application selon la revendication 11, 12 ou 13, caractérisée en ce que l'agent thiophile est la triphénylphosphine.

15. Application selon la revendication 11 ou 12, caractérisée en ce que l'agent oxydant est un peracide ou l'eau oxygénée en présence d'un acide carboxylique.

16. A titre de composés industriels, les composés de formule (IV), telle que définie à la revendication 11.

17. A titre de composés industriels, les composés de formule (V_{b}) : 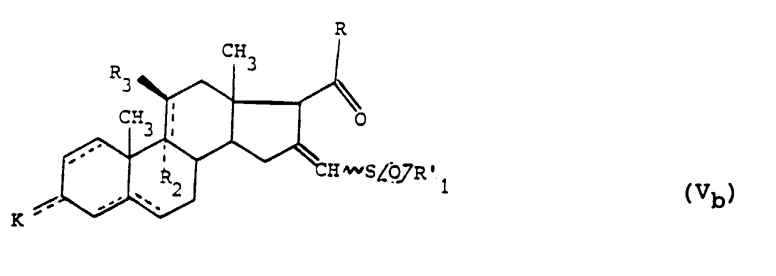 correspondant aux composés de formule (V), telle que définie à la revendication 11, dans laquelle le trait pointillé en position 16(17) et 16(16') représente une seconde liaison uniquement en position 16(16') ;
- les composés de formule (V_{c}) : 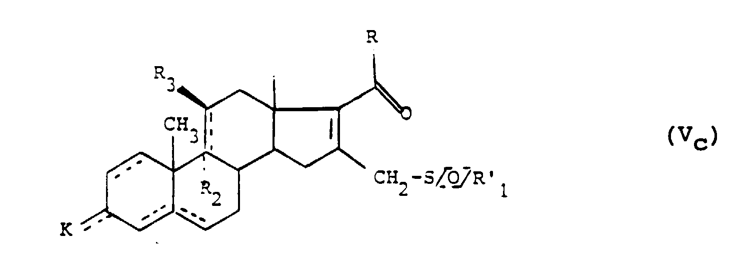 correspondant aux composés de formule (V), telle que définie à la revendication 11, dans laquelle le trait pointillé en position 16(17) et 16(16') représente une seconde liaison uniquement en position 16(17), à l'exception de ceux dans lesquels R représente un radical méthyle.

## Revendications (Revendications pour l'(les) Etat(s) contractant(s) suivant(s): ES)

1. Procédé pour préparer des composés de formule (A) : dans laquelle 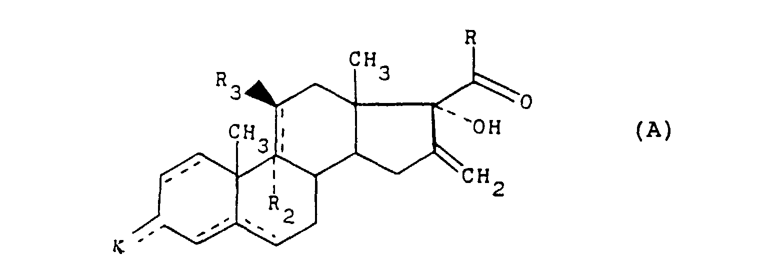 dans laquelle 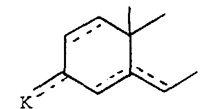 représente soit un système 3-céto Δ4 soit un système 3-céto Δ1,4 soit un système 3-OR₄ Δ5 dans lequel R₄ représente un atome d'hydrogène ou un groupement protecteur du radical hydroxy, R représente un radical méthyle, un radical -CH₂OH ou un radical -CH₂-OR', dans lequel R' représente un groupement protecteur du radical hydroxy, R₂ et R₃ représentent un atome d'hydrogène ou R₂ représente un atome de fluor et R₃ représente un radical formyloxy ou acétyloxy et les traits pointillés en position 9(11) symbolisent la présence éventuelle d'une seconde liaison, caractérisé en ce que l'on soumet un composé de formule (II) : 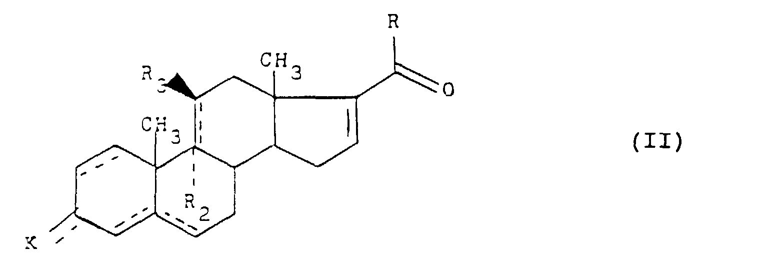 dans laquelle K, R, R₂, R₃ et les traits pointillés ont la signification indiquée ci-dessus, en milieu basique, à l'action d'un réactif de formule (P) :
R₁S-CH₂NO₂ (P)
dans laquelle R₁ représente un radical méthyle, un radical alkyle ramifié ne possédant pas d'atome d'hydrogène en position béta, renfermant de 5 à 8 atomes de carbone, un radical aryle renfermant jusqu'à 10 atomes de carbone, un radical hétéroaryle renfermant jusqu'à 10 atomes de carbone et un ou plusieurs hétéroatomes choisis parmi l'azote, le soufre et l'oxygène, ou un radical benzyle, pour obtenir un composé de formule (III) : 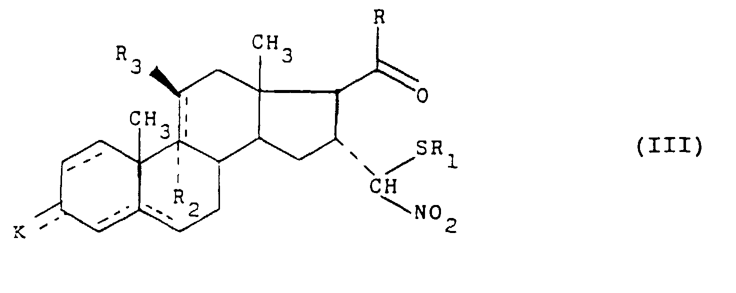 dans laquelle K, R, R₁, R₂, R₃ et les traits pointillés ont la signification indiquée plus haut, que l'on soumet, en présence d'un acide, à l'action d'un thiol ou d'un thiophénol de formule :
HS-R'₁
dans laquelle R'₁ identique ou différent de R₁ a la signification indiquée ci-dessus pour R₁, pour obtenir un composé de formule (I_{A}) : 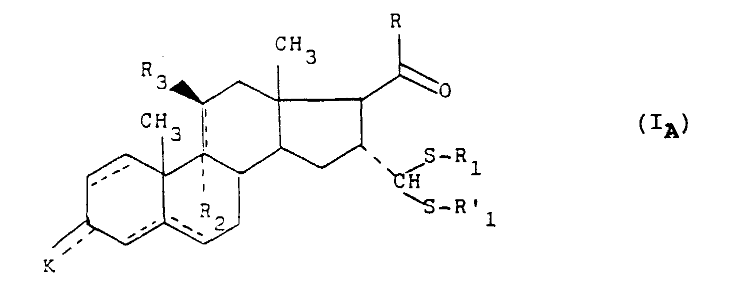 dans laquelle K, R, R₁, R'₁, R₂, R₃ et les traits pointillés ont la signification précitée, composé de formule (I_{A}) que, soit l'on soumet à l'action d'un équivalent d'un agent oxydant, pour obtenir un composé de formule (I_{B}) : 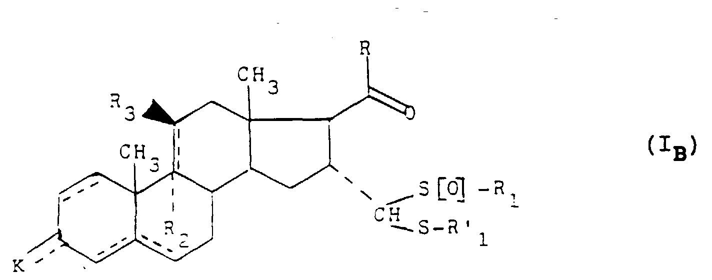 dans laquelle K, R, R₁, R'₁, R₂, R₃ et les traits pointillés ont la signification précitée, composé de formule (I_{B}) que l'on chauffe le cas échéant en présence d'un agent thiophile, pour obtenir un composé de formule (IV) : 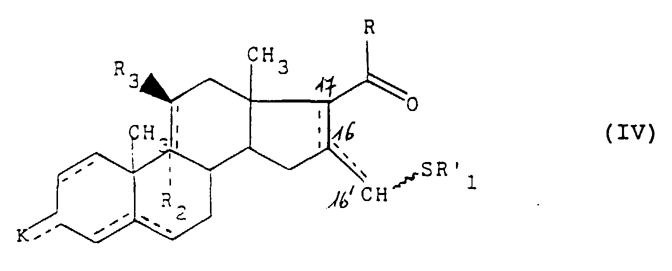 dans laquelle K, R, R'₁, R₂, R₃ et les traits pointillés ont la signification indiquée ci-dessus, le trait pointillé en 16(17) et 16(16') symbolise l'existence d'un mélange de sulfure vinylique et allylique et le trait ondulé symbolise l'existence d'un mélange d'isomères, composé de formule (IV) que l'on soumet à l'action d'au moins un équivalent d'un agent oxydant, pour obtenir un composé de formule (V) : 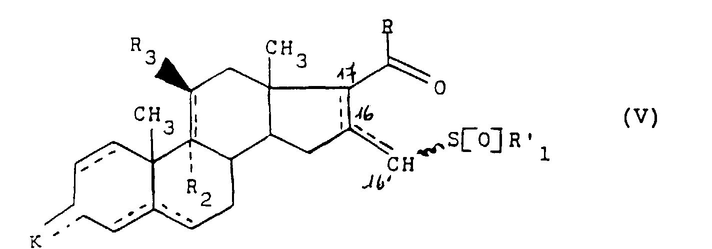 dans laquelle K, R, R'₁, R₂, R₃ les traits pointillés et le trait ondulé ont la signification indiquée ci-dessus, que l'on soumet, à chaud, à l'action d'un agent thiophile, pour obtenir le composé de formule (A) attendu,
soit l'on soumet à l'action d'au moins deux équivalents d'un agent oxydant, pour obtenir un composé de formule (I_{c}) : 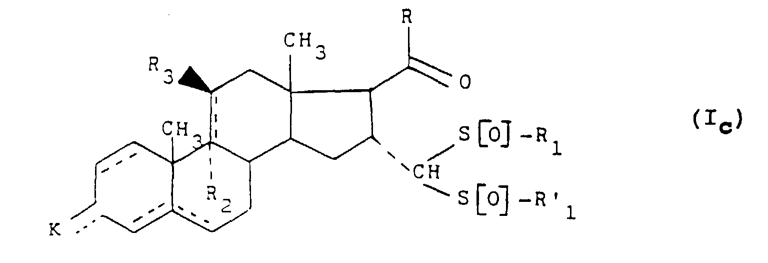 dans laquelle K, R, R₁, R'₁, R₂, R₃ et les traits pointillés ont la signification précitée, que l'on soumet, à chaud, à l'action d'un agent thiophile, pour obtenir le composé de formule (A) attendu.

2. Procédé selon la revendication 1, caractérisé en ce que l'on soumet un composé de formule (I_{A}) telle que définie à la revendication 1, à l'action d'au moins deux équivalents d'un agent oxydant, pour obtenir un composé de formule (I_{c}) telle que définie à la revendication 1, que l'on soumet, à chaud, à l'action d'un agent thiophile, pour obtenir le composé de formule (A) attendu.

3. Procédé selon la revendication 1 ou 2, caractérisé en ce que :
- l'action du réactif de formule (P) sur le composé de formule (II) est effectuée en présence d'une base aminée ;
- l'action du thiol ou du thiophénol est catalysée par un acide carboxylique ;
- l'agent oxydant est un peracide ou l'eau oxygénée en présence d'un acide carboxylique,
- l'agent thiophile est la triphénylphosphine.

4. Procédé selon la revendication 1, 2 ou 3, caractérisé en ce que l'on utilise au départ un composé de formule (II) dans laquelle R représente un radical méthyle, un radical -CH₂OH ou un radical -CH₂OR" dans lequel R" représente un radical alkyle renfermant de 1 à 4 atomes de carbone, un radical acyle renfermant de 1 à 5 atomes de carbone ou un radical benzyle, un réatif de formule (P) et un thiol ou thiophénol de formule HS-R'₁ tels que R₁ et R'₁, identiques ou différents, représentent un radical méthyle, phényle, tolyle ou benzyle.

5. Procédé selon l'une quelconque des revendications 1 à 4, caractérisé en ce que l'on utilise au départ un composé de formule (II) dans laquelle 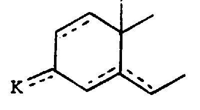 représente un système 3-céto Δ1,4 et R représente un radical -CH₂-OR", R" étant défini comme à la revendication 4, R₃ représente un atome d'hydrogène et les traits pointillés en position 9(11) représentent une seconde liaison.

6. Procédé selon l'une quelconque des revendications 1 à 5, caractérisé en ce que l'on choisit le composé de formule (II) et les réactifs de manière telle que l'on prépare :
- la 21-acétoxy 16alpha-[bis(phénylthio)-méthyl] pregna-1,4,9(11)triène-3,20-dione,
- la 21-acétoxy 16alpha-[(phénylthio) (phénylsulfinyl)-méthyl] pregna-1,4,9(11)-triène-3,20-dione,
- la 21-acétoxy 16alpha-[bis(phénylsulfinyl)-méthyl] pregna-1,4,9(11)-triène-3,20-dione,
et poursuit la synthèse comme indiqué à la revendication 1 pour obtenir la 21-acétoxy 16-méthylène 17α-hydroxy pregna 1,4,9(11)-triène 3,20-dione.

## Patentansprüche

1. Verbindungen der Formel (I): 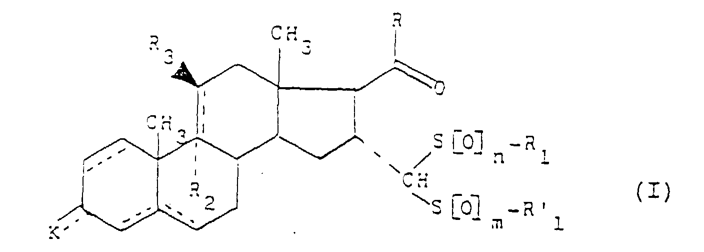 in der 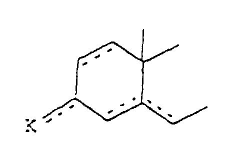 entweder ein 3-Keto-Δ4-System oder ein 3-Keto-Δ1,4-System oder ein 3-OR₄-Δ5-System darstellt, worin R₄ ein Wasserstoffatom oder eine Schutzgruppe des Hydroxylrestes darstellt, R einen Methylrest, einen Rest -CH₂OH oder einen Rest -CH₂-OR' darstellt, in dem R' eine Schutzgruppe des Hydroxylrestes darstellt, R₁ und R'₁, identisch oder verschieden, einen Methylrest, einen verzweigten Alkylrest, der kein Wasserstoffatom in der beta-Position besitzt und 5 bis 8 Kohlenstoffatome einschließt, einen Arylrest, der bis zu 10 Kohlenstoffatome einschließt, einen Heteroarylrest, der bis zu 10 Kohlenstoffatome und ein oder mehrere Heteroatome, ausgewählt aus Stickstoff, Schwefel und Sauerstoff, einschließt, oder einen Benzylrest darstellen, n und m, identisch oder verschieden, die Zahlen 0 oder 1 darstellen, R₂ und R₃ ein Wasserstoffatom darstellen oder R₂ ein Fluoratom darstellt und R₃ einen Formyloxy- oder Acetyloxyrest darstellt und die gestrichelten Linien in Position 9 (11) die mögliche Anwesenheit einer zweiten Bindung symbolisieren.

2. Verbindungen der Formel (I) wie in Anspruch 1 definiert, in denen R einen Methylrest, einen Rest -CH₂OH oder einen Rest -CH₂OR" darstellt, worin R" einen Alkylrest mit 1 bis 4 Kohlenstoffatomen, einen Acylrest mit 1 bis 5 Kohlenstoffatomen oder einen Benzylrest darstellt, R₁ und R'₁, identisch oder verschieden, einen Methyl-, Phenyl-, Tolyl- oder Benzylrest darstellen, wobei n, m, R₂, R₃ und die gestrichelten Linien wie in Anspruch 1 definiert sind.

3. Verbindungen der Formel (I), wie in Anspruch 1 oder 2 definiert, die der Formel (I_{A}) entsprechen: 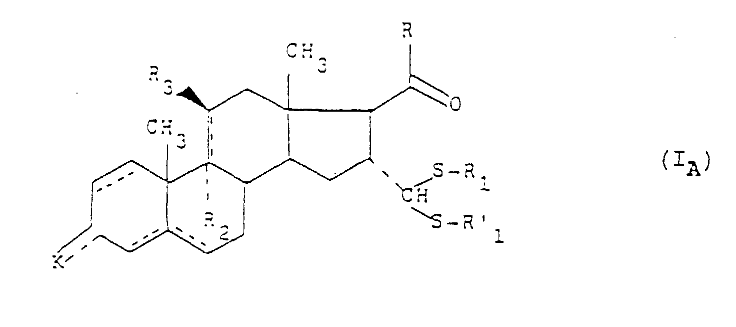 in der K, R, R₁, R'₁, R₂, R₃ und die gestrichelten Linien wie vorstehend definiert sind.

4. Verbindungen der Formel (I), wie in Anspruch 1 oder 2 definiert, die der Formel (I_{B}) entsprechen: 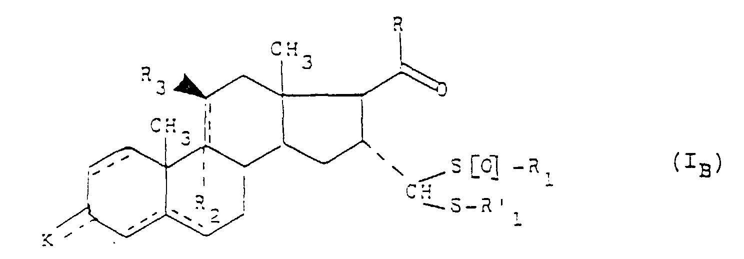 in der K, R, R₁, R'₁, R₂, R₃ und die gestrichelten Linien wie vorstehend definiert sind.

5. Verbindungen der Formel (I), wie in Anspruch 1 oder 2 definiert, die der Formel (I_{C}) entsprechen: 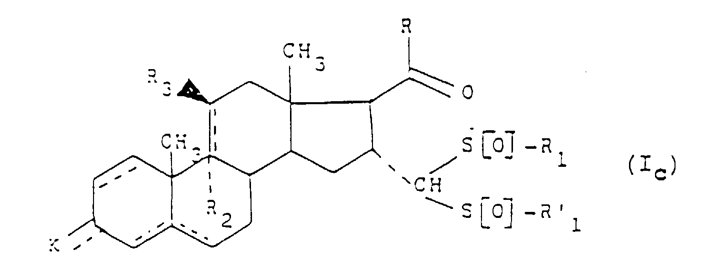 in der K, R, R₁, R'₁, R₂, R₃ und die gestrichelten Linien wie in Anspruch 1 oder 2 definiert sind.

6. Verbindungen der Formel (I), wie in irgendeinem der Ansprüche 1 bis 5 definiert, dadurch gekennzeichnet, daß 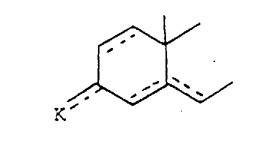 ein 3-Keto-Δ1,4-System darstellt und R einen Rest -CH₂-OR" darstellt, wobei R" wie in Anspruch 2 definiert ist, R₃ ein Wasserstoffatom darstellt und die gestrichelten Linien in Position 9 (11) eine zweite Bindung darstellen.

7. Verfahren zur Herstellung von Verbindungen der Formel (I), wie in Anspruch 1 definiert, dadurch gekennzeichnet, daß man eine Verbindung der Formel (II): 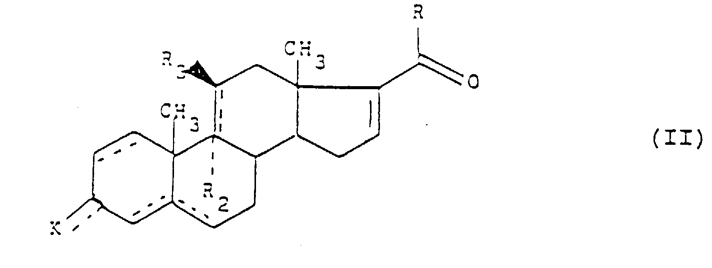 in der K, R, R₂, R₃ und die gestrichelten Linien die oben angegebene Bedeutung aufweisen, in einem basischen Medium der Einwirkung eines Reagenz der Formel (P) unterzieht:
R₁S-CH₂-NO₂ (P)
in der R₁ die oben angegebene Bedeutung aufweist, um eine Verbindung der Formel (III) zu erhalten: 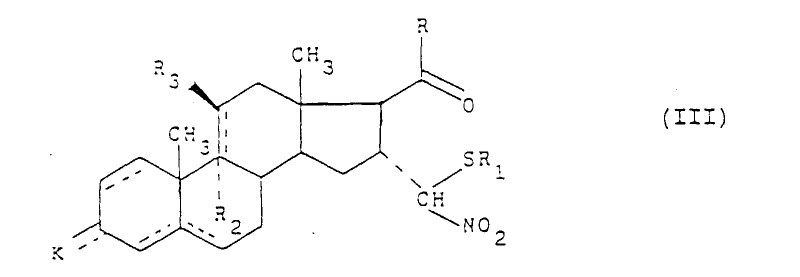 in der K, R, R₁, R₂, R₃ und die gestrichelten Linien die oben angegebene Bedeutung aufweisen, welche man in Anwesenheit einer Säure der Einwirkung eines Thiols oder eines Thiophenols der Formel:
HS-R'₁
unterzieht, in der R'₁ die vorstehende Bedeutung aufweist, um eine Verbindung der Formel (I_{A}) zu erhalten: 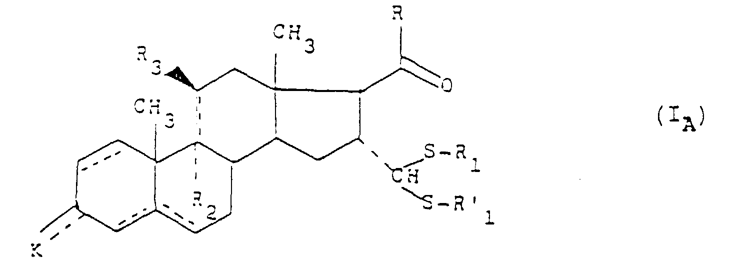 in der K, R, R₁, R'₁, R₂, R₃ und die gestrichelten Linien die vorstehende Bedeutung aufweisen, die einer Verbindung der Formel (I) entspricht, in der m und n gleich 0 sind, daß man die Verbindung der Formel (I_{A}) gegebenenfalls entweder der Einwirkung eines Äquivalents eines Oxidationsmittels unterzieht, um eine Verbindung der Formel (I_{B}) zu erhalten: 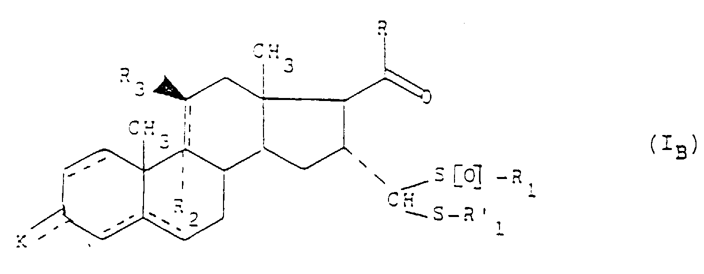 in der K, R, R₁, R'₁, R₂, R₃ und die gestrichelten Linien die vorstehende Bedeutung aufweisen, welche einer Verbindung der Formel (I) entspricht, in der einer der Werte m und n gleich 1 ist und der andere gleich 0 ist, oder der Einwirkung von mindestens zwei Äquivalenten eines Oxidationsmittels unterzieht, um eine Verbindung der Formel (I_{C}) zu erhalten: 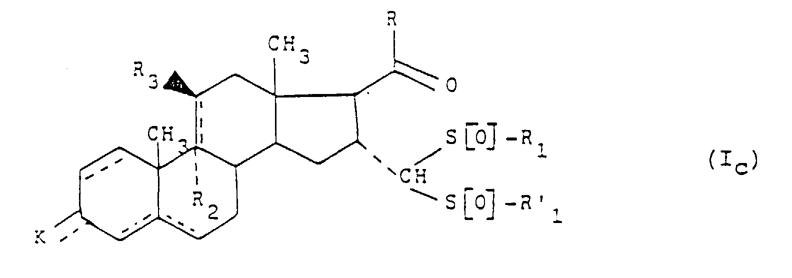 in der K, R, R₁, R'₁, R₂, R₃ und die gestrichelten Linien die vorstehende Bedeutung aufweisen, welche einer Verbindung der Formel (I) entspricht, in der m und n gleich 1 sind.

8. Verfahren nach Anspruch 7, dadurch gekennzeichnet, daß man eine Verbindung der Formel (II): 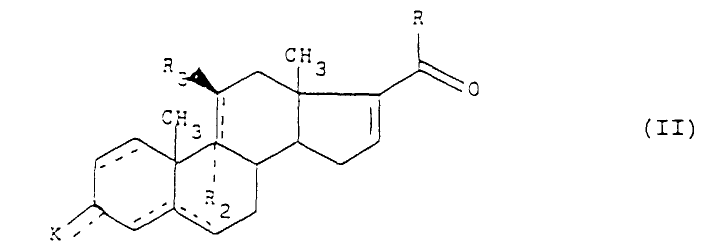 in der K, R, R₂, R₃ und die gestrichelten Linien die oben angegebene Bedeutung aufweisen, in einem basischen Medium der Einwirkung eines Reagenz der Formel (P) unterzieht:
R₁S-CH₂-NO₂ (P)
in der R₁ die oben angegebene Bedeutung aufweist, um eine Verbindung der Formel (III) zu erhalten: 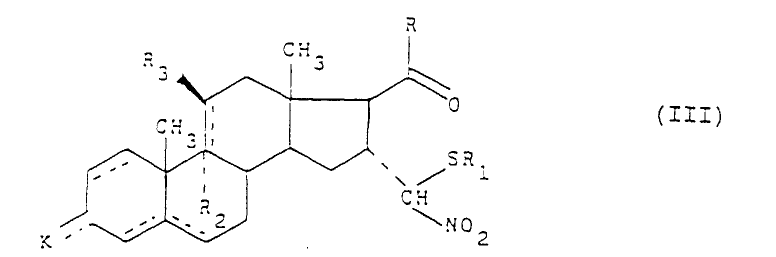 in der K, R, R₁, R₂, R₃ und die gestrichelten Linien die oben angegebene Bedeutung aufweisen, welche man in Anwesenheit einer Säure der Einwirkung eines Thiols oder eines Thiophenols der Formel:
HS-R'₁
unterzieht, in der R'₁ die vorstehende Bedeutung aufweist, um eine Verbindung der Formel (I_{A}) zu erhalten: 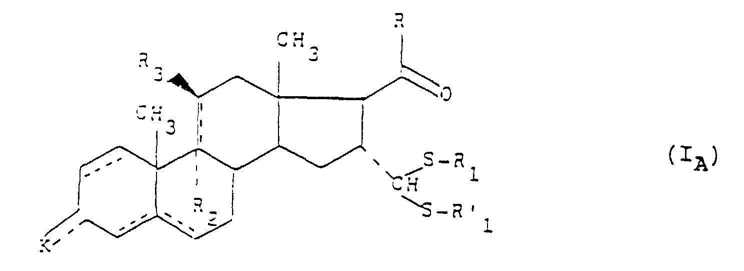 in der K, R, R₁, R'₁, R₂, R₃ und die gestrichelten Linien die vorstehende Bedeutung aufweisen, welche einer Verbindung der Formel (I) entspricht, in der m und n gleich 0 sind, daß man die Verbindung der Formel (I_{A}) der Einwirkung von mindestens zwei Äquivalenten eines Oxidationsmittels unterzieht, um eine Verbindung der Formel (I_{C}) zu erhalten: 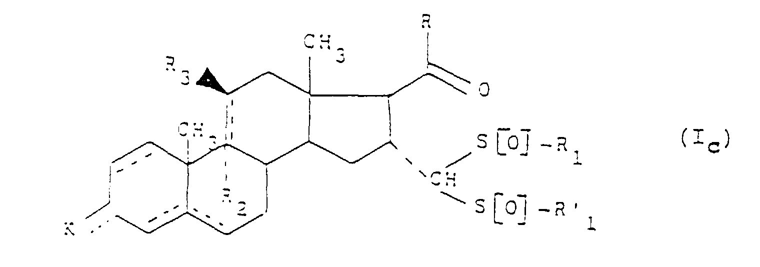 in der K, R, R₁, R'₁, R₂, R₃ und die gestrichelten Linien die vorstehende Bedeutung aufweisen, welche einer Verbindung der Formel (I) entspricht, in der m und n gleich 1 sind.

9. Verfahren nach Anspruch 7 oder 8, dadurch gekennzeichnet, daß:
- die Einwirkung des Reagenz der Formel (P) auf die Verbindung der Formel (II) in Anwesenheit einer aminierten Base durchgeführt wird;
- die Einwirkung des Thiols oder des Thiophenols durch eine Carbonsäure katalysiert wird;
- das Oxidationsmittel eine Persäure oder Wasserstoffperoxid in Anwesenheit einer Carbonsäure ist.

10. Verbindungen der Formel (III), wie in Anspruch 7 definiert, als neue industrielle Verbindungen.

11. Verwendung von Verbindungen der Formel (I), wie in Anspruch 1 definiert, bei der Herstellung von Verbindungen der Formel (A): 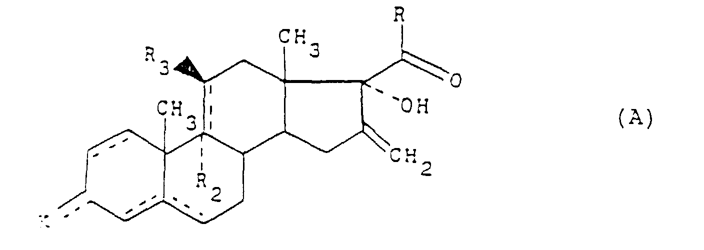 in der K, R, R₂, R₃ und die gestrichelten Linien die in Anspruch 1 angegebene Bedeutung aufweisen, dadurch gekennzeichnet, daß man:
- entweder eine Verbindung der Formel (I_{A}), wie in Anspruch 3 definiert, der Einwirkung von mindestens zwei Äquivalenten eines Oxidationsmittels unterzieht, um eine Verbindung der Formel (I_{C}) zu erhalten, wie in Anspruch 5 definiert, die man in der Wärme der Einwirkung eines thiophilen Mittels unterzieht, um die erwartete Verbindung der Formel (A) zu erhalten;
- oder eine Verbindung der Formel (I_{C}), wie in Anspruch 5 definiert, in der Wärme der Einwirkung eines thiophilen Mittels unterzieht, um die erwartete Verbindung der Formel (A) zu erhalten;
- oder eine Verbindung der Formel (I_{B}), wie in Anspruch 4 definiert, gegebenenfalls in Anwesenheit eines thiophilen Mittels erwärmt, um eine Verbindung der Formel (IV) zu erhalten: 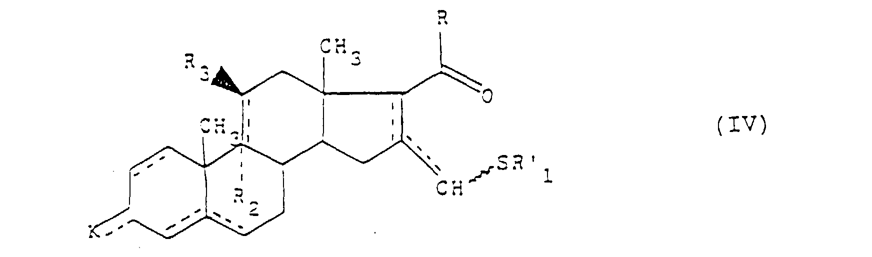 in der K, R, R'₁, R₂, R₃ und die gestrichelten Linien die in Anspruch 1 angegebene Bedeutung aufweisen, die gestrichelte Linie in 16 (17) und 16 (16') die Anwesenheit einer Mischung von vinylischem und allylischem Schwefel symbolisiert und die Wellenlinie die Anwesenheit einer Mischung von Isomeren symbolisiert, daß man die Verbindung der Formel (IV) der Einwirkung von mindestens einem Äquivalent eines Oxidationsmittels unterzieht, um eine Verbindung der Formel (V): 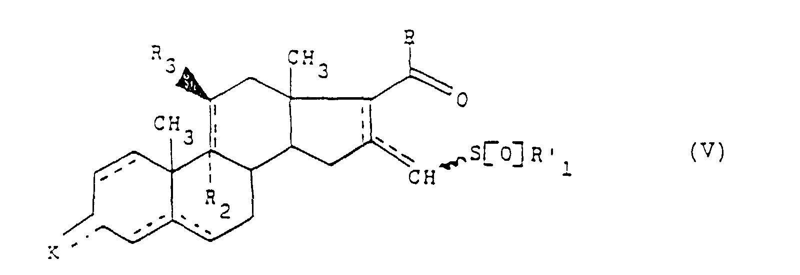 zu erhalten, in der K, R, R'₁, R₂, R₃, die gestrichelten Linien und die Wellenlinie die oben angegebene Bedeutung aufweisen, welche man in der Wärme der Einwirkung eines thiophilen Mittels unterzieht, um die erwartete Verbindung der Formel (A) zu erhalten.

12. Verwendung nach Anspruch 11, dadurch gekennzeichnet, daß man eine Verbindung der Formel (I_{A}), wie in Anspruch 3 definiert, der Einwirkung von mindestens zwei Äquivalenten eines Oxidationsmittels unterzieht, um eine Verbindung der Formel (I_{C}), wie in Anspruch 5 definiert, zu erhalten, die man in der Wärme der Einwirkung eines thiophilen Mittels unterzieht, um die erwartete Verbindung der Formel (A) zu erhalten.

13. Verwendung nach Anspruch 11, dadurch gekennzeichnet, daß man eine Verbindung der Formel (I_{C}), wie in Anspruch 5 definiert, in der Wärme der Einwirkung eines thiophilen Mittels unterzieht, um die erwartete Verbindung der Formel (A) zu erhalten.

14. Verwendung nach Anspruch 11, 12 oder 13, dadurch gekennzeichnet, daß es sich bei dem thiophilen Mittel um Triphenylphosphin handelt.

15. Verwendung nach Anspruch 11 oder 12, dadurch gekennzeichnet, daß das Oxidationsmittel eine Persäure oder Wasserstoffperoxid in Anwesenheit einer Carbonsäure ist.

16. Verbindungen der Formel (IV), wie in Anspruch 11 definiert, als industrielle Verbindungen.

17. Verbindungen der Formel (V_{b}): 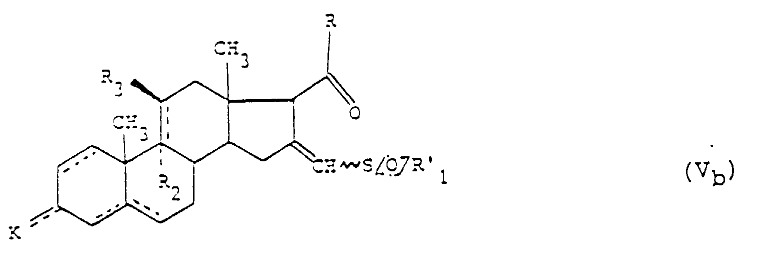 die den Verbindungen der Formel (V), wie in Anspruch 11 definiert, entsprechen, in der die gestrichelte Linie in Position 16 (17) und 16 (16') eine zweite Bindung allein in Position 16 (16') darstellt;
Verbindungen der Formel (V_{c}): 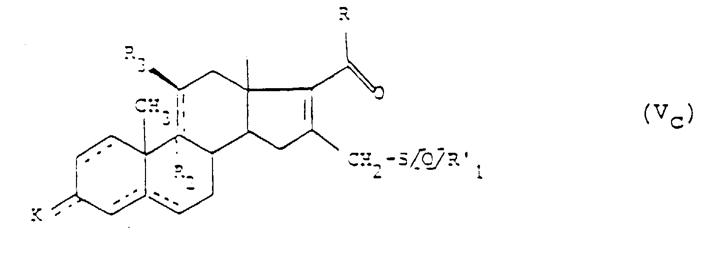 die den Verbindungen der Formel (V), wie in Anspruch 11 definiert, entsprechen, in der die gestrichelte Linie in Position 16 (17) und 16 (16') eine zweite Bindung allein in Position 16 (17) darstellt, mit Ausnahme derjenigen, in denen R einen Methylrest darstellt,
als industrielle Verbindungen.

## Patentansprüche (Patentansprüche für folgende(n) Vertragsstaat(en): ES)

1. Verfahren zur Herstellung von Verbindungen der Formel (A): 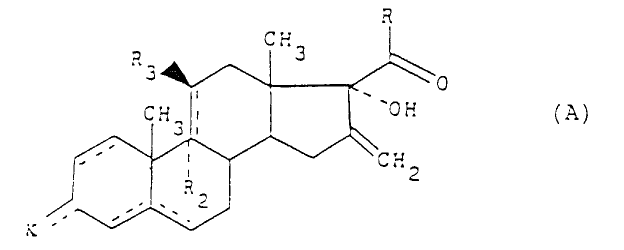 in der 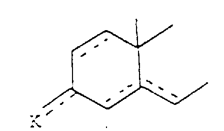 entweder ein 3-Keto-Δ4-System oder ein 3-Keto-Δ1,4-System oder ein 3-OR₄-Δ5-System darstellt, worin R₄ ein Wasserstoffatom oder eine Schutzgruppe des Hydroxylrestes darstellt, R einen Methylrest, einen Rest -CH₂OH oder einen Rest -CH₂-OR' darstellt, in dem R' eine Schutzgruppe des Hydroxylrestes darstellt, R₂ und R₃ ein Wasserstoffatom darstellen oder R₂ ein Fluoratom darstellt und R₃ einen Formyloxy- oder Acetyloxyrest darstellt und die gestrichelten Linien in Position 9 (11) die mögliche Anwesenheit einer zweiten Bindung symbolisieren, dadurch gekennzeichnet, daß man eine Verbindung der Formel (II): 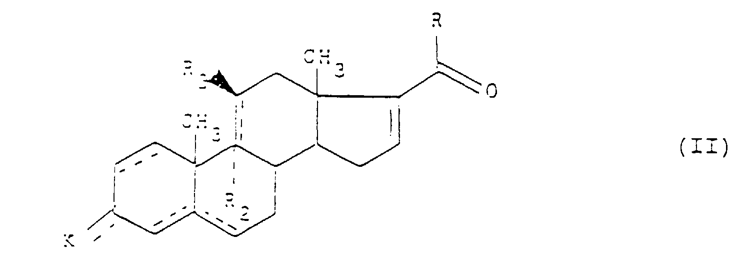 in der K, R, R₂, R₃ und die gestrichelten Linien die oben angegebene Bedeutung aufweisen, in einem basischem Medium der Einwirkung eines Reagenz der Formel (P) unterzieht:
R₁S-CH₂-NO₂ (P)
in der R₁ einen Methylrest, einen verzweigten Alkylrest, der kein Wasserstoffatom in beta-Position besitzt und 5 bis 8 Kohlenstoffatome einschließt, einen Arylrest, der bis zu 10 Kohlenstoffatome einschließt, einen Heteroarylrest, der bis zu 10 Kohlenstoffatome und ein oder mehrere Heteroatome, ausgewählt aus Stickstoff, Schwefel und Sauerstoff, einschließt, oder einen Benzylrest bedeutet, um eine Verbindung der Formel (III) zu erhalten: 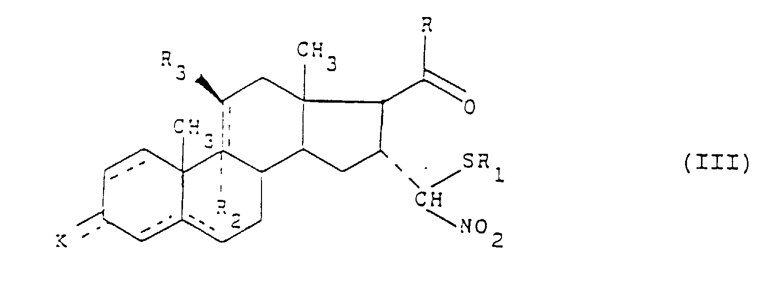 in der K, R, R₁, R₂, R₃ und die gestrichelten Linien die oben angegebene Bedeutung aufweisen, welche man in Anwesenheit einer Säure der Einwirkung eines Thiols oder eines Thiophenols der Formel:
HS-R'₁
unterzieht, in der R'₁, identisch oder verschieden von R₁, die oben angegebene Bedeutung für R₁ aufweist, um eine Verbindung der Formel (I_{A}) zu erhalten: 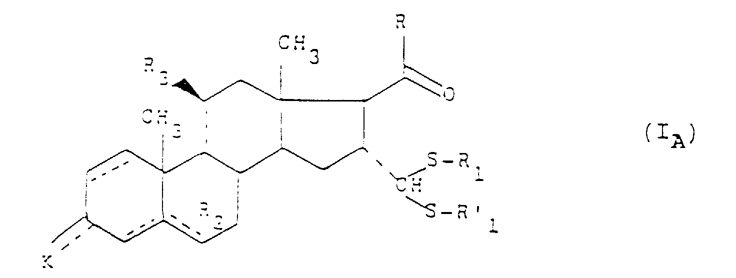 in der K, R, R₁, R'₁, R₂, R₃ und die gestrichelten Linien die vorstehende Bedeutung aufweisen, daß man die Verbindung der Formel (I_{A}) entweder der Einwirkung eines Äquivalents eines Oxidationsmittels unterzieht, um eine Verbindung der Formel (I_{B}) zu erhalten: 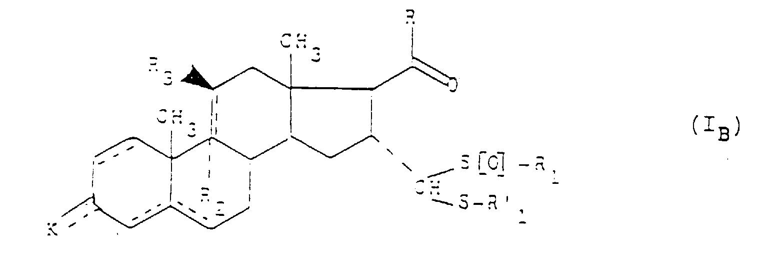 in der K, R, R₁, R'₁, R₂, R₃ und die gestrichelten Linien die vorstehende Bedeutung aufweisen, daß man die Verbindung der Formel (I_{B}) gegebenenfalls in Anwesenheit eines thiophilen Mittels erwärmt, um eine Verbindung der Formel (IV) zu erhalten: 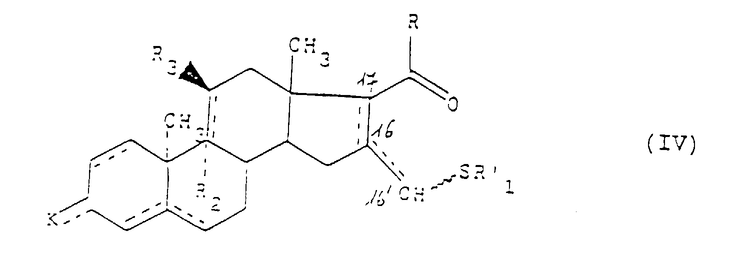 in der K, R, R'₁, R₂, R₃ und die gestrichelten Linien die oben angegebene Bedeutung aufweisen, die gestrichelte Linie in 16 (17) und 16 (16') die Anwesenheit einer Mischung von vinylischem und allylischem Schwefel symbolisiert und die Wellenlinie die Anwesenheit einer Mischung von Isomeren symbolisiert, daß man die Verbindung der Formel (IV) der Einwirkung von mindestens einem Äquivalent eines Oxidationsmittels unterzieht, um eine Verbindung der Formel (V) zu erhalten: 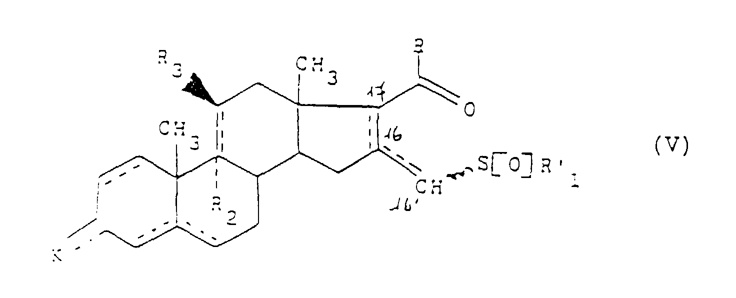 in der K, R, R'₁, R₂, R₃, die gestrichelten Linien und die Wellenlinie die oben angegebene Bedeutung aufweisen, die man in der Wärme der Einwirkung eines thiophilen Mittels unterzieht, um die erwartete Verbindung der Formel (A) zu erhalten,
oder daß man sie der Einwirkung von mindestens zwei Äquivalenten eines Oxidationsmittels unterzieht, um eine Verbindung der Formel (I_{C}) zu erhalten: 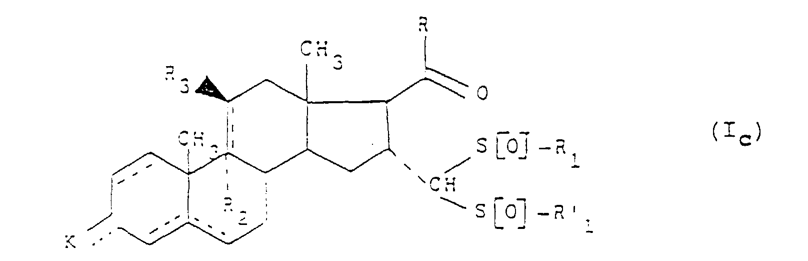 in der K, R, R₁, R'₁, R₂, R₃ und die gestrichelten Linien die vorstehende Bedeutung aufweisen, die man in der Wärme der Einwirkung eines thiophilen Mittels unterzieht, um die erwartete Verbindung der Formel (A) zu erhalten.

2. Verfahren nach Anspruch 1, dadurch gekennzeichnet, daß man eine Verbindung der Formel (I_{A}), wie in Anspruch 1 definiert, der Einwirkung von mindestens zwei Äquivalenten eines Oxidationsmittels unterzieht, um eine Verbindung der Formel (I_{C}), wie in Anspruch 1 definiert, zu erhalten, die man in der Wärme der Einwirkung eines thiophilen Mittels unterzieht, um die erwartete Verbindung der Formel (A) zu erhalten.

3. Verfahren nach Anspruch 1 oder 2, dadurch gekennzeichnet, daß:
- die Einwirkung des Reagenz der Formel (P) auf die Verbindung der Formel (II) in Anwesenheit einer aminierten Base durchgeführt wird;
- die Einwirkung des Thiols oder des Thiophenols durch eine Carbonsäure katalysiert wird;
- das Oxidationsmittel eine Persäure oder Wasserstoffperoxid in Anwesenheit einer Carbonsäure ist;
- das thiophile Mittel Triphenylphosphin ist.

4. Verfahren nach Anspruch 1, 2 oder 3, dadurch gekennzeichnet, daß man zu Beginn eine Verbindung der Formel (II), in der R einen Methylrest, einen Rest-CH₂OH oder einen Rest -CH₂OR" darstellt, worin R" einen Alkylrest mit 1 bis 4 Kohlenstoffatomen, einen Acylrest mit 1 bis 5 Kohlenstoffatomen oder einen Benzylrest darstellt, ein Reagenz der Formel (P) und ein Thiol oder ein Thiophenol der Formel HS-R'₁ verwendet, so daß R₁ und R'₁, identisch oder verschieden, einen Methyl-, Phenyl-, Tolyl- oder Benzylrest darstellen.

5. Verfahren nach irgendeinem der Ansprüche 1 bis 4, dadurch gekennzeichnet, daß man zu Beginn eine Verbindung der Formel (II) verwendet, in der 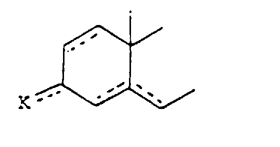 ein 3-Keto-Δ1,4-System darstellt und R einen Rest -CH₂-OR" darstellt, wobei R" wie in Anspruch 4 definiert ist, R₃ ein Wasserstoffatom darstellt und die gestrichelten Linien in Position 9 (11) eine zweite Bindung darstellen.

6. Verfahren nach irgendeinem der Ansprüche 1 bis 5, dadurch gekennzeichnet, daß man die Verbindung der Formel (II) und die Reagenzien derart wählt, daß man herstellt:
- 21-Acetoxy-16alpha-[bis(phenylthio)methyl]pregna-1,4,9(11)-trien-3,20-dion,
- 21-Acetoxy-16alpha-[(phenylthio)(phenylsulfinyl)methyl]pregna-1,4,9(11)-trien-3,20-dion,
- 21-Acetoxy-16a1pha-[bis(phenylsulfinyl)methyl]pregna-1,4,9(11)-trien-3,20-dion,
und die Synthese wie in Anspruch 1 angegeben weiter verfolgt, um 21-Acetoxy-16-methylen-17α-hydroxypregna-1,4,9(11)-trien-3,20-dion zu erhalten.

## Claims

1. The compounds of formula (I): 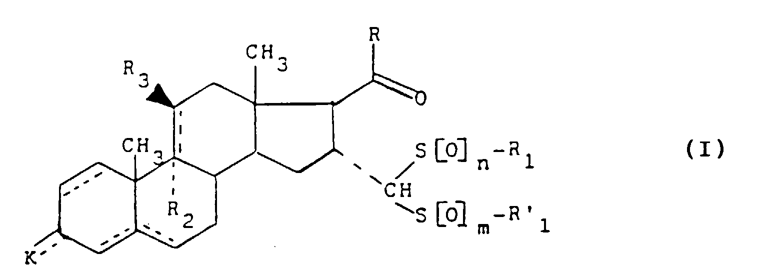 in which 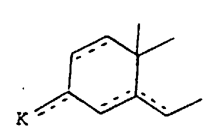 represents either a 3-ketoΔ4 system or a 3-keto Δ1,4 system or a 3-OR₄ Δ5 system in which R₄ represents a hydrogen atom or a protective group of the hydroxy radical, R represents a methyl radical, a -CH₂OH radical or a -CH₂-OR' radical, in which R' represents a protective group of the hydroxy radical, R₁ and R'₁, identical or different, represent a methyl radical, a branched alkyl radical not possessing a hydrogen atom in the beta position, containing 5 to 8 carbon atoms, an aryl radical containing up to 10 carbon atoms, a heteroaryl radical containing up to 10 carbon atoms and one or more heteroatoms chosen from nitrogen, sulphur and oxygen, or a benzyl radical, n and m, identical or different, represent the numbers 0 or 1, R₂ and R₃ represent a hydrogen atom or R₂ represents a fluorine atom and R₃ represents a formyloxy or acetyloxy radical and the dotted lines in position 9(11) symbolize the optional presence of a second bond.

2. The compounds of formula (I), as defined in claim 1, in which R represents a methyl radical, a -CH₂OH radical or a -CH₂OR" radical in which R" represents an alkyl radical containing 1 to 4 carbon atoms, an acyl radical containing 1 to 5 carbon atoms or a benzyl radical, R₁ and R'₁, identical or different, represent a methyl, phenyl, tolyl or benzyl radical, n, m, R₂, R₃ and the dotted lines being defined as in claim 1.

3. The compounds of formula (I) as defined in claim 1 or 2, corresponding to formula (I_{A}): 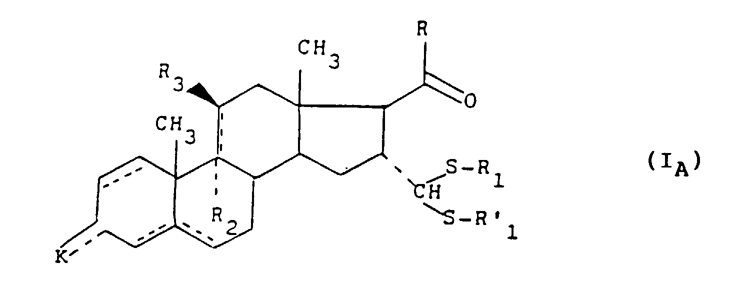 in which K, R, R₁, R'₁, R₂, R₃ and the dotted lines are defined as previously,

4. The compounds of formula (I), as defined in claim 1 or 2, corresponding to formula (I_{B}): 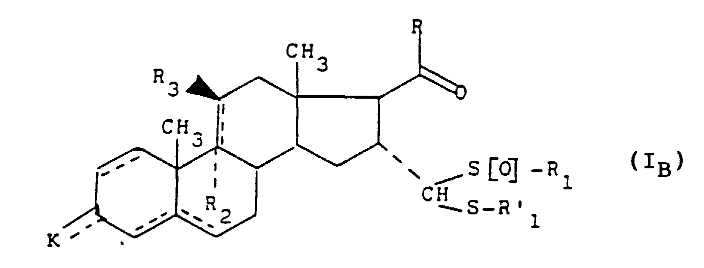 in which K, R, R₁, R'₁, R₂, R₃ and the dotted lines are defined as previously.

5. The compounds of formula (I), as defined in claim 1 or 2, corresponding to formula (I_{C}): 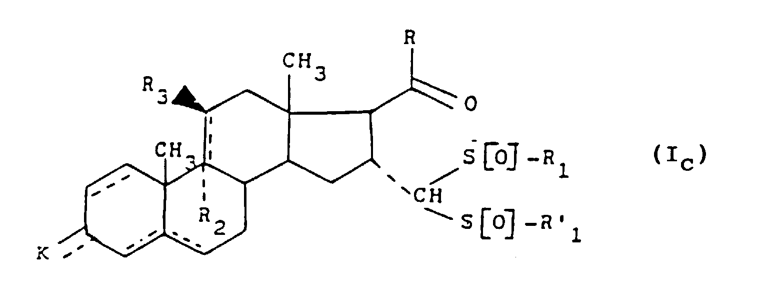 in which K, R, R₁, R'₁, R₂, R₃ and the dotted lines are defined as in claim 1 or 2.

6. The compounds of formula (I), as defined in any one of claims 1 to 5, characterized in that 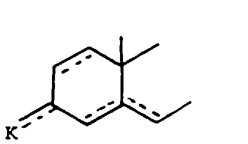 represents a 3-ketoΔ1,4 system and R represents a -CH₂-OR" radical, R" being defined as in claim 2, R₃ represent a hydrogen atom and the dotted lines in position 9(11) represent a second bond.

7. Preparation process for compounds of formula (I), as defined in claim 1, characterized in that a compound of formula (II): 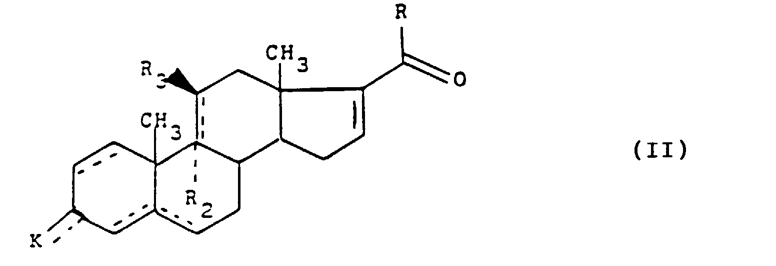 in which K, R, R₂, R₃ and the dotted lines have the meaning indicated above is subjected, in a basic medium, to the action of a reagent of formula (P):
R₁S-CH₂-NO₂ (P)
in which R₁ has the meaning indicated above, in order to obtain a compound of formula (III): 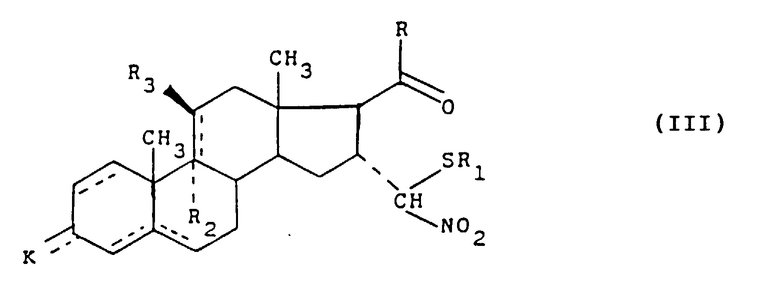 in which K, R, R₁, R₂, R₃ and the dotted lines have the meaning indicated above, which is subjected, in the presence of an acid, to the action of a thiol or a thiophenol of formula:
HS-R'₁
in which R'₁ has the meaning mentioned previously, in order to obtain a compound of formula (I_{A}): 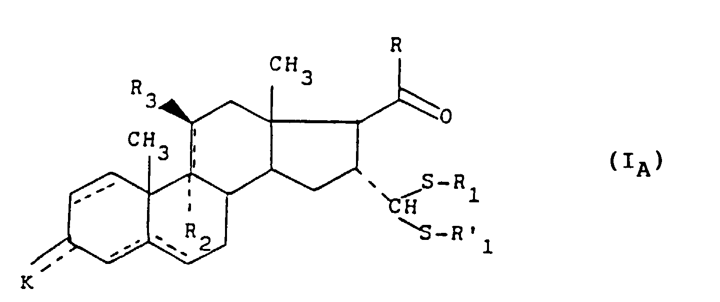 in which K, R, R₁, R'₁, R₂, R₃ and the dotted lines have the meaning mentioned previously, corresponding to a compound of formula (I) in which m and n are equal to 0, which compound of formula (I_{A}), if appropriate, is subjected either to the action of an equivalent of an oxidizing agent, in order to obtain a compound of formula (I_{B}): 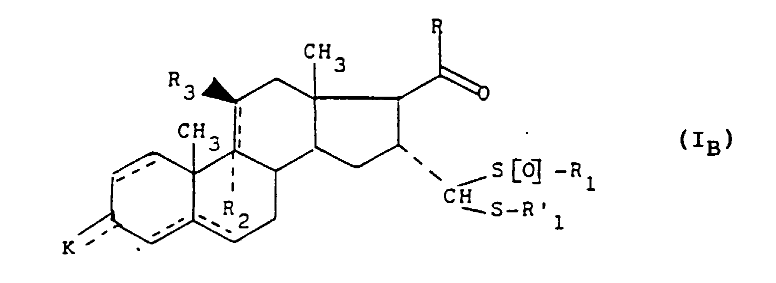 in which K, R, R₁, R'₁, R₂, R₃ and the dotted lines have the meaning mentioned previously, corresponding to a compound of formula (I) in which one of the values m and n is equal to 1 and the other to 0, or to the action of at least two equivalents of an oxidizing agent, in order to obtain a compound of formula (I_{C}): 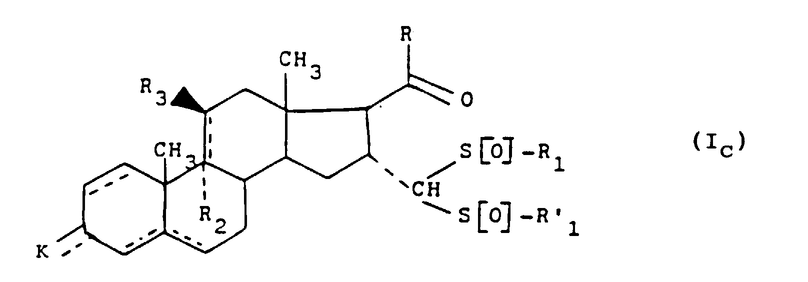 in which K, R, R₁, R'₁, R₂, R₃ and the dotted lines have the meaning mentioned previously, corresponding to a compound of formula (I) in which m and n are equal to 1.

8. Process according to claim 7, characterized in that a compound of formula (II): 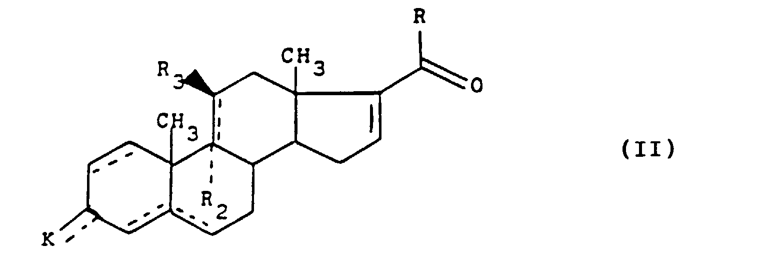 in which K, R, R₂, R₃ and the dotted lines have the meaning indicated above, is subjected, in a basic medium, to the action of a reagent of formula (P):
R₁S-CH₂-NO₂ (P)
in which R₁ has the meaning indicated above, in order to obtain a compound of formula (III): 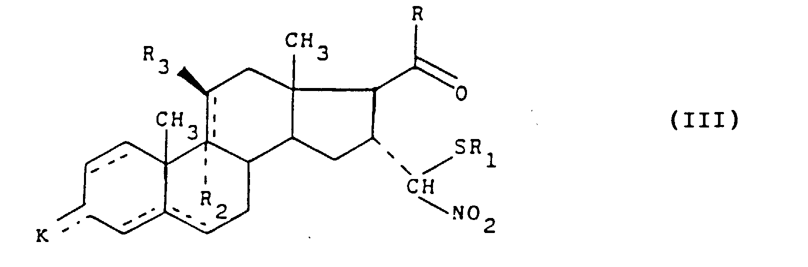 in which K, R, R₁, R₂, R₃ and the dotted lines have the meaning indicated above, which is subjected in the presence of an acid to the action of a thiol or a thiophenol of formula:
HS-R'₁
in which R'₁ has the meaning mentioned previously, in order to obtain a compound of formula (I_{A}): 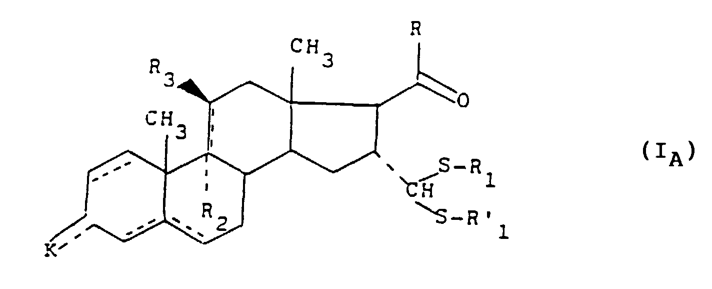 in which K, R, R₁, R'₁, R₂, R₃ and the dotted lines have the meaning mentioned previously, corresponding to a compound of formula (I) in which m and n are equal to 0, which compound of formula (I_{A}) is subjected to the action of at least two equivalents of an oxidizing agent, in order to obtain a compound of formula (I_{C}): 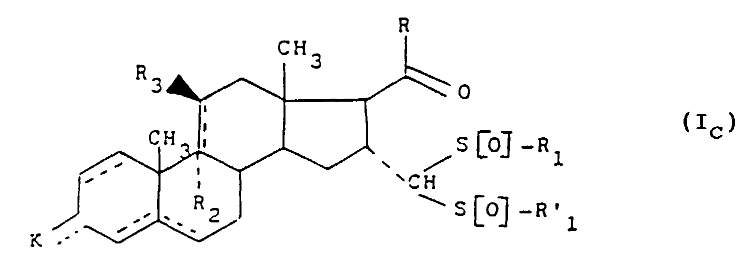 in which K, R, R₁, R'₁, R₂, R₃ and the dotted lines have the meaning mentioned previously, corresponding to a compound of formula (I) in which m and n are equal to 1.

9. Process according to claim 7 or 8, characterized in that:
- the action of the reagent of formula (P) on the compound of formula (II) is carried out in the presence of an amino base;
- the action of the thiol or the thiophenol is catalyzed by a carboxylic acid;
- the oxidizing agent is a peracid or hydrogen peroxide in the presence of a carboxylic acid.

10. As new industrial compounds, the compounds of formula (III), as defined in claim 7.

11. Use of the compounds of formula (I), as defined in claim 1, for the preparation of compounds of formula (A): 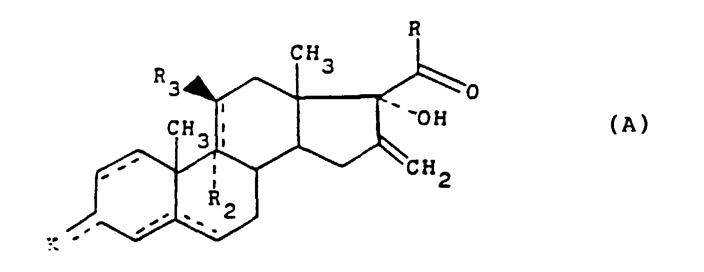 in which K, R, R₂, R₃ and the dotted lines have the meaning indicated in claim 1, characterized in that:
- either a compound of formula (I_{A}) as defined in claim 3 is subjected to the action of at least two equivalents of an oxidizing agent, in order to obtain a compound of formula (I_{C}) as defined in claim 5, which is subjected, warm, to the action of a thiophilic agent, in order to obtain the expected compound of formula (A);
- or a compound of formula (I_{C}) as defined in claim 5 is subjected, warm, to the action of a thiophilic agent, in order to obtain the expected compound of formula (A);
- or a compound of formula (I_{B}) as defined in claim 4 is heated, if appropriate in the presence of a thiophilic agent, in order to obtain a compound of formula (IV): 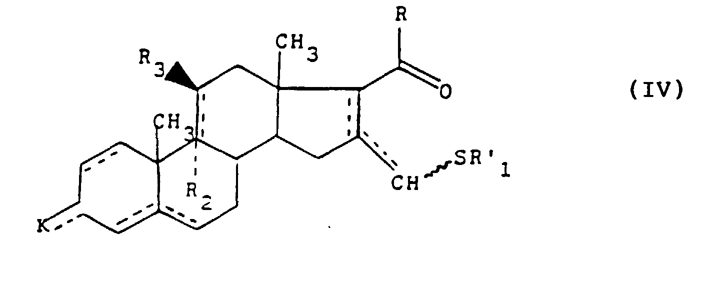 in which K, R, R'₁, R₂, R₃ and the dotted lines have the meaning indicated in claim 1, the dotted line in position 16(17) and 16(16') symbolizes the existence of a vinyl and allyl sulphide mixture and the wavy line symbolizes the existence of an isomer mixture, which compound of formula (IV) is subjected to the action of at least one equivalent of an oxidizing agent, in order to obtain a compound of formula (V): 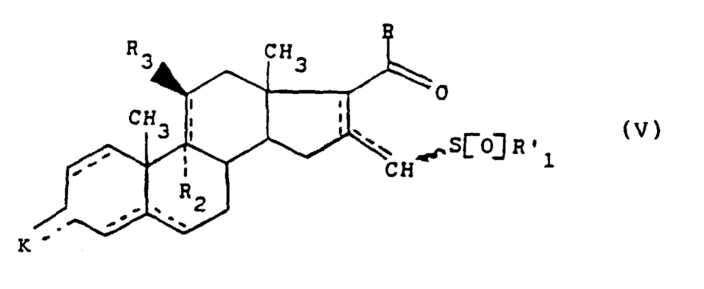 in which K, R, R'₁, R₂, R₃, the dotted lines and the wavy line have the meaning indicated above, which is subjected, warm, to the action of a thiophilic agent, in order to obtain the expected compound of formula (A).

12. Use according to claim 11, characterized in that a compound of formula (I_{A}) as defined in claim 3 is subjected to the action of at least two equivalents of an oxidizing agent, in order to obtain a compound of formula (I_{C}) as defined in claim 5, which is subjected, warm, to the action of a thiophilic agent, in order to obtain the expected compound of formula (A).

13. Use according to claim 11, characterized in that a compound of formula (I_{C}) as defined in claim 5 is subjected, warm, to the action of a thiophilic agent, in order to obtain the expected compound of formula (A).

14. Use according to claim 11, 12 or 13, characterized in that the thiophilic agent is triphenylphosphine.

15. Use according to claim 11 or 12, characterized in that the oxidizing agent is a peracid or hydrogen peroxide in the presence of a carboxylic acid.

16. As new industrial compounds, the compounds of formula (IV), as defined in claim 11.

17. As new industrial compounds, the compounds of formula (V_{b}): 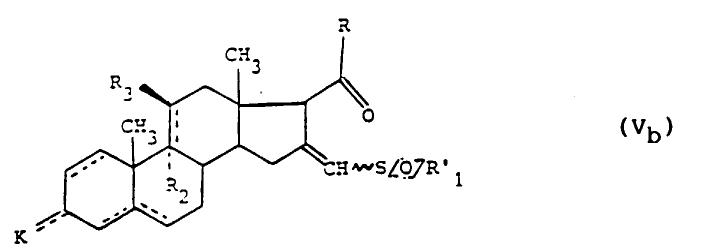 corresponding to the compounds of formula (V), as defined in claim 11, in which the dotted line in position 16(17) and 16(16') represents a second bond only in position 16(16');
- the compounds of formula (V_{c}): 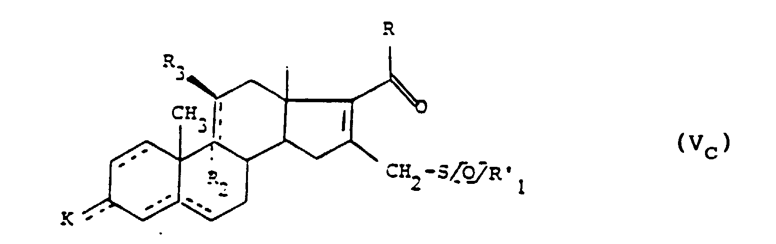 corresponding to the compounds of formula (V), as defined in claim 11, in which the dotted line in position 16(17) and 16(16') represents a second bond only in position 16(17), with the exception of those in which R represents a methyl radical.

## Claims (Claims for the following Contracting State(s): ES)

1. Preparation process for the compounds of formula (A): 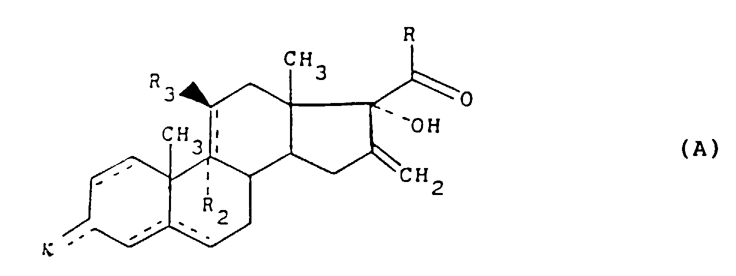 in which 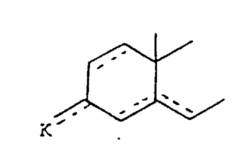 represents either a 3-keto Δ4 system or a 3-ketoΔ1,4 system or a 3-OR₄ Δ5 system in which R₄ represents a hydrogen atom or a protective group of the hydroxy radical, R represents a methyl radical, a -CH₂OH radical or a -CH₂-OR' radical, in which R' represents a protective group of the hydroxy radical, R₂ and R₃ represent a hydrogen atom or R₂ represents a fluorine atom and R₃ represents a formyloxy or acetyloxy radical and the dotted lines in position 9(11) symbolize the optional presence of a second bond, characterized in that a compound of formula (II): 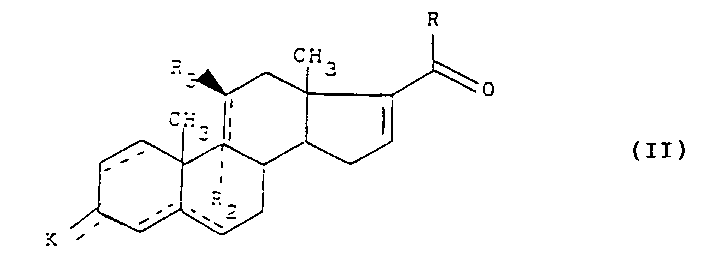 in which K, R, R₂, R₃ and the dotted lines have the meaning indicated above is subjected, in a basic medium, to the action of a reagent of formula (P):
R₁S-CH₂-NO₂ (P)
in which R₁ represents a methyl radical, a branched alkyl radical not possessing a hydrogen atom in the beta position, containing 5 to 8 carbon atoms, an aryl radical containing up to 10 carbon atoms, a heteroaryl radical containing up to 10 carbon atoms and one or more heteroatoms chosen from nitrogen, sulphur and oxygen, or a benzyl radical, in order to obtain a compound of formula (III): 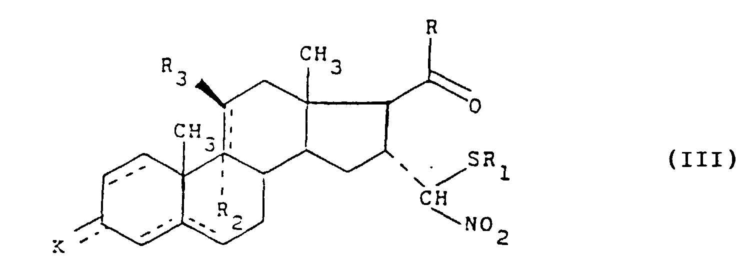 in which K, R, R₁, R₂, R₃ and the dotted lines have the meaning indicated above, which is subjected, in the presence of an acid, to the action of a thiol or a thiophenol of formula:
HS-R'₁
in which R'₁ identical to or different from R₁ has the meaning indicated above for R₁, in order to obtain a compound of formula (I_{A}): 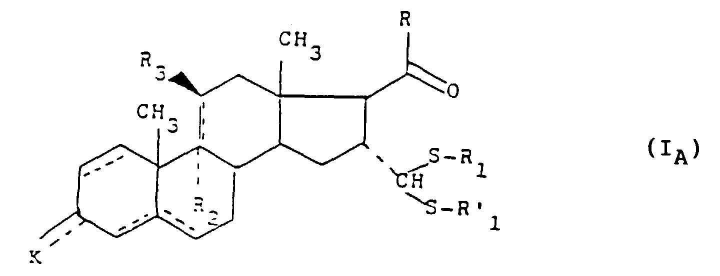 in which K, R, R₁, R'₁, R₂, R₃ and the dotted lines have the meaning mentioned previously, which compound of formula (I_{A}), either is subjected to the action of an equivalent of an oxidizing agent, in order to obtain a compound of formula (I_{B}): 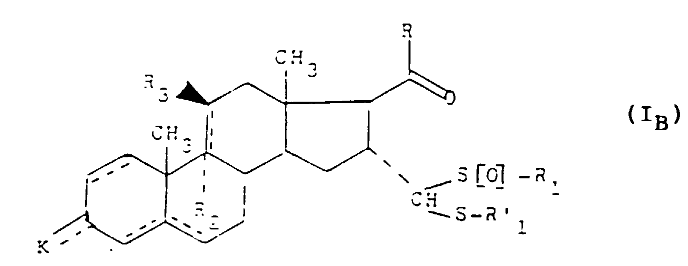 in which K, R, R₁, R'₁, R₂, R₃ and the dotted lines have the meaning mentioned previously, which compound of formula (I_{B}) is heated, if appropriate in the presence of a thiophilic agent, in order to obtain a compound of formula (IV): 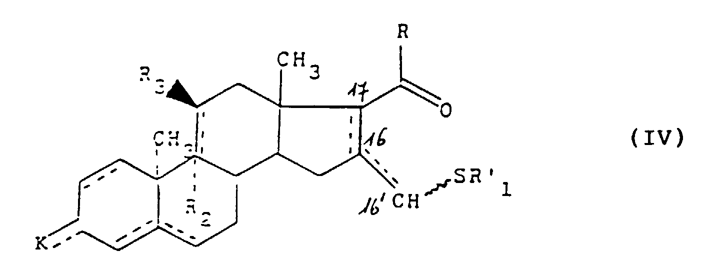 in which K, R, R'₁, R₂, R₃ and the dotted lines have the meaning indicated above, the dotted line in position 16(17) and 16(16') symbolizes the existence of a vinyl and allyl sulphide mixture and the wavy line symbolizes the existence of an isomer mixture, which compound of formula (IV) is subjected to the action of at least one equivalent of an oxidizing agent, in order to obtain a compound of formula (V): 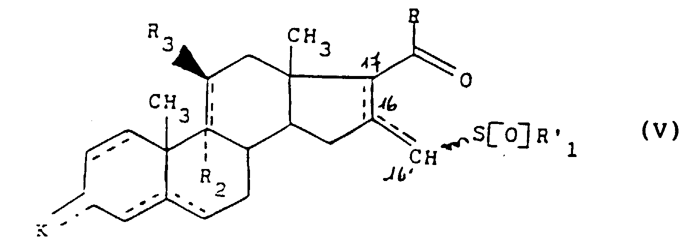 in which K, R, R'₁, R₂, R₃, the dotted lines and the wavy line have the meaning indicated above, which is subjected, warm, to the action of a thiophilic agent, in order to obtain the expected compound of formula (A),
or is subjected to the action of at least two equivalents of an oxidizing agent, in order to obtain a compound of formula (I_{c}): 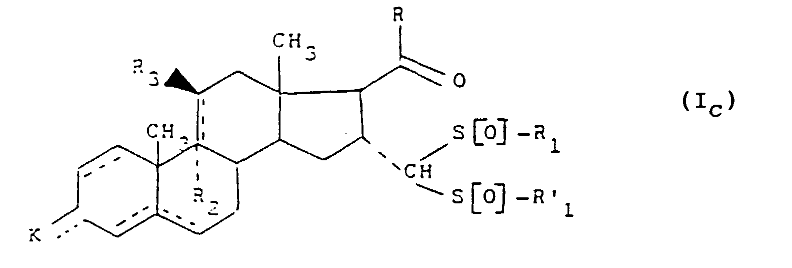 in which K, R, R₁, R'₁, R₂, R₃ and the dotted lines have the meaning mentioned previously, which is subjected, warm, to the action of a thiophilic agent, in order to obtain the expected compound of formula (A).

2. Process according to claim 1, characterized in that a compound of formula (I_{A}) as defined in claim 1, is subjected to the action of at least two equivalents of an oxidizing agent, in order to obtain a compound of formula (I_{c}) as defined in claim 1, which is subjected, warm, to the action of a thiophilic agent, in order to obtain the expected compound of formula (A).

3. Process according to claim 1 or 2, characterized in that:
- the action of the reagent of formula (P) on the compound of formula (II) is carried out in the presence of an amino base;
- the action of the thiol or the thiophenol is catalyzed by a carboxylic acid;
- the oxidizing agent is a peracid or hydrogen peroxide in the presence of a carboxylic acid;
- the thiophilic agent is triphenylphosphine.

4. Process according to claim 1, 2 or 3, characterized in that a compound of formula (II) is used at the start in which R represents a methyl radical, a -CH₂OH radical or a -CH₂OR" radical in which R" represents an alkyl radical containing 1 to 4 carbon atoms, an acyl radical containing 1 to 5 carbon atoms or a benzyl radical, a reagent of formula (P) and a thiol or thiophenol of formula HS-R'₁ such that R₁ and R'₁, identical or different, represent a methyl, phenyl, tolyl or benzyl radical.

5. Process according to any one of claims 1 to 4, characterized in that a compound of formula (II) is used at the start in which 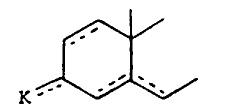 represents a 3-ketoΔ1,4 system and R represents a -CH₂-OR" radical, R" being defined as in claim 4, R₃ represents a hydrogen atom and the dotted lines in position 9(11) represent a second bond.

6. Process according to any one of claims 1 to 5, characterized in that the compound of formula (II) and the reagents are chosen in such a way that the following are prepared:
- 21-acetoxy-16alpha-[bis(phenylthio)-methyl] pregna-1,4,9(11)-triene-3,20-dione,
- 21-acetoxy-16alpha-[(phenylthio) (phenylsulphinyl)methyl] pregna-1,4,9(11)-triene-3,20-dione
- 21-acetoxy-16alpha-[bis(phenylsulphinyl)-methyl] pregna-1,4,9(11)-triene-3,20-dione,
and the synthesis is continued as indicated in claim 1 in order to obtain 21-acetoxy 16-methylene 17alpha-hydroxy pregna 1,4,9(11)-triene 3,20-dione.
